# EUROPÄISCHE PATENTSCHRIFT

(11) **EP 1 107 968 B1**
(45) Veröffentlichungstag und Bekanntmachung des Hinweises auf die Patenterteilung: **13.11.2002**
(21) Anmeldenummer: 99941615.9
(22) Anmeldetag: 13.08.1999
(51) Int. Cl.: C07D 487/04, A61K 31/519, A61P 9/00, A61P 15/00

(54) **DIHYDRO-[1,2,3]TRIAZOLO-[4,5-D]PYRIMIDIN-7-ON**
DIHYDRO-[1,2,3]TRIAZOLO-[4,5-D]PYRIMIDIN-7-ONE
DIHYDRO-[1,2,3]TRIAZOLO-[4,5-D]PYRIMIDINE-7-ONE

(30) Priorität: 26.08.1998 DE 19838705
(43) Veröffentlichungstag der Anmeldung: 20.06.2001
(73) Patentinhaber: BAYER AG, 51368 Leverkusen (DE)
(72) Erfinder: HANING, Helmut, D-42115 Wuppertal (DE); NIEWÖHNER, Ulrich, D-42929 Wermelskirchen (DE); ROSENTRETER, Ulrich, D-42349 Wuppertal (DE); SCHENKE, Thomas, D-51469 Bergisch Gladbach (DE); BISCHOFF, Erwin, D-42115 Wuppertal (DE); SCHLEMMER, Karl-Heinz, D-42113 Wuppertal (DE)
(86) Internationale Anmeldenummer: EP9905955
(87) Internationale Veröffentlichungsnummer: WO00012504

(56) Entgegenhaltungen:
- EP-A- 0 771 799
- WO-A-96/28429
- GB-A- 1 338 235
- US-A- 4 076 711

## Beschreibung

Die vorliegende Erfindung betrifft neue Dihydro-[1,2,3]triazolo-[4,5-d]pyrimidin-7-on, Verfahren zu ihrer Herstellung und ihre Verwendung als Arzneimittel, insbesondere als Inhibitoren cGMP-metabolisierender Phosphodiesterasen.

Die Synthese von Triazolopyrimidinen wurde in J. Chem. Soc., C, 1971, 706 beschrieben. Fungizide Eigenschaften von Dihydrotriazolopyrimidinonen und deren Synthese wurden in Liebigs Ann. 1984, 1848 von Nielsen et. al. beschrieben.

Die Synthese von Dihydrotriazolpyrimidinonen durch Cyclisierung eines Esters mit einem Aminotriazolocarbonamid in Gegenwart einer Base wurde in J. Het. Chem. 1985, 22, 1607 von Biagi et al und in Het. 1996, 42, 691 von Miyashita et al. beschrieben; die analoge Synthese ausgehend von Amidinen findet sich in J. Chem. Soc., Perkin Trans. 1, 1979, 922.

Antiallergische Eigenschaften von 1-unsubstituierten Dihydrotriazolopyrimidinonen wurden in Eur. J. Med. Chem. 1975, 447, in J. Med. Chem. 1975, 1117 und in J. Am. Chem. Soc. 1978, 100, 6474 beschrieben. Die Synthese von 5-Amino-dihydrotriazolopyrimidinonen wurde in J. Chem. Soc. 1961, 4433 beschrieben und über deren antivirale Aktivität wurde in J. Med. Chem. 1987, 30, 1091 und J. Med. Chem. 1984, 27, 1416 berichtet.

Über Anticytokine Eigenschaften von 8-Azaadeninen wird von Karanov et al. in Plant Growth Regul. 1993, 13, 7 berichtet.

Eine Synthesemethode für Azahypoxanthine und Azaadenine wird von Biagi et al, in J. Het. Chem. 1991, 28, 1351 und J. Het. Chem. 1989, 26, 39 beschrieben.

Antikonvulsive Eigenschaften von 5-Amino-Dihydrotriazolopyrimidinonen wurde in EP-288 431 beschrieben. Die Synthese von 5-Amino-Dihydrotriazolopyrimidinonen wird weiterhin in J. Het. Chem. 1996, 33, 1605 beschrieben. Über Adenosin A1 Rezeptoraffinität von Azaadeninen und Bindung zu Adenosindeaminase wurde in Farmaco 1996, 51, 395, Farmaco 1995, 50, 659 und Farmaco 1994, 49, 187 berichtet.

Purin-Nucleosid Phosphorylase inhibitorische Eigenschaften von Azaguaninen werden in J. Med. Chem. 1996, 39, 949 beschrieben.

Synthesen von Dihydrotriazolopyrimidinonen finden sich weiterhin in Farmaco 1995, 50, 13, Farmaco 1994, 49, 183, Farmaco 1992, 47, 1457, Farmaco 1992, 47, 525 und in Chem. Ztg. 1990, 114, 246.

Die Synthese von Zwischenprodukten des Typs II wurde in EP 0 229 011 und in J. Chem. Soc. 78, 1956, 5832 beschrieben.

Triazolopyrimidinonen der allgemeinen Formel (I) mit dem angegebenen Substitutionsmuster R_{I}, A, D und L sind neu.

Die erfindungsgemäßen Verbindungen sind potente Inhibitoren von entweder einer oder mehrere der cyclischen Guanosin 3',5'-monophophat metabolisierenden Phosphodiesterasen (cGMP -PDE's). Entsprechend der Nomenklatur von Beavo und Reifsnyder (Trends in Pharmacol. Sci. 11, 150-155, 1990) handelt es sich um die Phosphodiesterase Isoenzyme PDE-I, PDE-II und PDE-V.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen. Es kann die Kurz- oder Langzeitmodulation der vaskulären und kardialen Inotropie, den Herzrhythmus und die kardiale Erregungsleitung beeinflussen (J.C. Stoclet, T. Keravis, N. Komas and C. Kugnier, Exp. Opin. Invest. Drugs (1995), 4 (11), 1081-1100).

Die vorliegende Erfindung betrifft jetzt Dihydro-[1,2,3]triazolo-[4,5-d]pyrimidin-7-one der allgemeinen Formel (I) 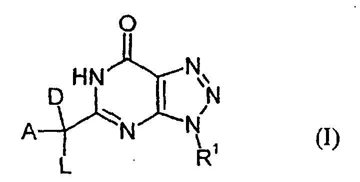 in welcher
- R¹: für Cycloalkyl mit 3 bis 8 Kohlenstoffatomen steht, oder
für einen Rest der Formel 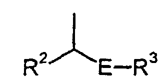 steht,
worin
- R²: geradkettiges oder verzweigtes Alkyl mit bis zu 10 Kohlenstoffatomen bedeutet, das gegebenenfalls durch Hydroxy substituiert ist,
- E: einen Rest der Formel -CH₂-T bedeutet,
worin
- T: eine geradkettige oder verzweigte Alkylenkette mit bis zu 10 Kohlenstoffatomen bedeutet,
- R³: Wasserstoff oder Aryl mit 6 bis 10 Kohlenstoffatomen bedeutet, das gegebenenfalls bis zu 3-fach gleich oder verschieden durch Halogen, Hydroxy, Nitro, Trifluormethyl oder durch geradkettiges oder verzweigtes Alkyl oder Alkoxy mit jeweils bis zu 6 Kohlenstoffatomen substituiert ist,
- A und D: für Wasserstoff stehen,
oder
- A: für Wasserstoff
und
- D: für Hydroxy steht,
oder
- A und D: gemeinsam für einen Rest der Formel =O stehen,
- L: für einen Rest der Formel 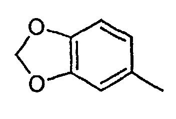 steht, oder
für Aryl mit 6 bis 10 Kohlenstoffatomen oder für einen 5- bis 7-gliedrigen aromatischen, gegebenenfalls benzokondensierten Heterocyclus mit bis zu 3 Heteroatomen aus der Reihe S, N und/oder O steht, wobei die oben unter L aufgeführten Ringsysteme bis zu 3-fach gleich oder verschieden substituiert sind, durch einen oder mehreren der folgenden Substituenten: Halogen, Hydroxy, Nitro, Trifluormethyl, Carboxy, geradkettiges oder verzweigtes Alkyl, Alkoxy und Alkoxycarbonyl mit jeweils bis zu 6 Kohlenstoffatomen, einen Rest der Formel -(V)ₐ-NR⁴R⁵,
worin
- a: eine Zahl 0 oder 1 bedeutet,
- V: einen Rest der Formel -CO oder -SO₂ bedeutet,
- R⁴ und R⁵: gleich oder verschieden sind und Wasserstoff oder geradkettiges oder verzweigtes Acyl oder Alkoxycarbonyl mit jeweils bis zu 6 Kohlenstoffatomen bedeuten, oder
geradkettiges oder verzweigtes Alkyl mit bis zu 6 Kohlenstoffatomen bedeuten, das gegebenenfalls durch Hydroxy, Amino oder durch geradkettiges oder verzweigtes Alkyl- oder Dialkylamino mit jeweils bis zu 6 Kohlenstoffatomen substituiert ist, oder
- R⁴ und R⁵: gemeinsam mit dem Stickstoffatom einen 5- oder 6-gliedrigen, gesättigten Heterocyclus bilden, der gegebenenfalls ein weiteres Heteroatom aus der Reihe S, O oder einen Rest der Formel -NR⁶ enthalten kann und der gegebenenfalls durch geradkettiges oder verzweigtes Alkoxycarbonyl mit bis zu 6 Kohlenstoffatomen substituiert ist,
worin
- R⁶: Wasserstoff oder geradkettiges oder verzweigtes Alkyl mit bis zu 4 Kohlenstoffatomen bedeutet,
und/oder die unter L aufgeführten Ringsysteme gegebenenfalls durch Aryl mit 6 bis 10 Kohlenstoffatomen und einen 5- bis 7-gliedrigen aromatischen, gegebenenfalls benzokondensierten Heterocyclus mit bis zu 3 Heteroatomen aus der Reihe S, N und/oder O substituiert sind, wobei diese Ringsysteme ihrerseits gegebenenfalls bis zu 2-fach gleich oder verschieden durch Substituenten aus der Reihe Halogen, Hydroxy, Nitro, Carboxyl, Trifluormethyl oder durch geradkettiges oder verzweigtes Alkyl, Alkoxy oder Alkoxycarbonyl mit jeweils bis zu 5 Kohlenstoffatomen oder durch eine Gruppe der Formel -(V')_{b}-NR⁷R⁸ substituiert sind,
worin
- b: die oben angegebene Bedeutung von a hat und mit dieser gleich oder verschieden ist,
- R⁷ und R⁸: die oben angegebene Bedeutung von R⁴ und R⁵ haben und mit dieser gleich oder verschieden sind,
- V': die oben angegebene Bedeutung von V hat und mit dieser gleich oder verschieden ist,
und deren Tautomere und Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastcreomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Cycloalkyl mit 3 bis 8 Kohlenstoffatomen steht im Rahmen der Erfindung für Cyclopropyl, Cyclopentyl, Cyclobutyl, Cyclohexyl, Cycloheptyl oder Cyclooctyl. Bevorzugt seien genannt: Cyclopropyl, Cyclopentyl und Cyclohexyl.

Aryl mit 6 bis 10 Kohlenstoffatomen steht im allgemeinen für einen aromatischen Rest mit 6 bis 10 Kohlenstoffatomen. Bevorzugte Arylreste sind Phenyl und Naphthyl.

Alkyl mit bis zu 6 Kohlenstoffatomen steht im Rahmen der Erfindung für einen geradkettigen oder verzweigten Alkylrest mit 1 bis 6 Kohlenstoffatomen. Bevorzugt ist ein geradkettiger oder verzweigter Alkylrest mit 1 bis 4 Kohlenstoffatomen. Besonders bevorzugt ist ein geradkettiger oder verzweigter Alkylrest mit 1 bis 3 Kohlenstoffatomen. Beispielsweise seien genannt: Methyl, Ethyl, n-Propyl, Isopropyl, tert.Butyl, n-Pentyl und n-Hexyl.

Alkoxy mit bis zu 6 Kohlenstoffatomen steht im Rahmen der Erfindung für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen. Besonders bevorzugt ist ein geradkettiger oder verzweigter Alkoxyrest mit 1 bis 3 Kohlenstoffatomen. Beispielsweise seien genannt: Methoxy, Ethoxy, n-Propoxy, Isopropoxy, tert.Butoxy, n-Pentoxy und n-Hexoxy.

Alkoxycarbonyl mit bis zu 6 Kohlenstoffatomen steht im Rahmen der Erfindung für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen. Besonders bevorzugt ist ein geradkettiger oder verzweigter Alkoxycarbonylrest mit 1 bis 3 Kohlenstoffatomen. Beispielsweise seien genannt: Methoxycarbonyl, Ethoxycarbonyl, n-Propoxycarbonyl, Isopropoxycarbonyl und tert.Butoxycarbonyl.

Acyl mit bis zu 6 Kohlenstoffatomen steht im Rahmen der Erfindung für einen geradkettigen oder verzweigten Acylrest mit 1 bis 6 Kohlenstoffatomen. Bevorzugt ist ein geradkettiger oder verzweigter Acylrest mit 1 bis 4 Kohlenstoffatomen. Besonders bevorzugt ist ein geradkettiger oder verzweigter Acylrest mit 1 bis 3 Kohlenstoffatomen. Beispielsweise seien genannt: Acetoxy, Ethylcarbonyl oder n-Propylcarbonyl.

Heterocyclus steht im Rahmen der Erfindung in Abhängigkeit des jeweiligen Substituenten im allgemeinen für einen aromatischen, gegebenenfalls benzokondensierten oder ungesättigten 5- bis 7-gliedrigen, vorzugsweise 5- bis 6-gliedrigen Heterocyclus der bis zu 3 Heteroatome aus der Reihe S, N und/oder O enthalten kann, oder für einen 5- bis 6-gliedrigen, gesättigten Heterocyclus der gegebenenfalls ein weiteres Heteroatom aus der Reihe S oder O enthalten kann. Beispielsweise seien genannt: Pyridin, Pyrimidyl, Piperazinyl, Thienyl, Furyl, Morpholinyl, Pyrrolidinyl, Piperazinyl oder Piperidyl. Bevorzugt sind Pyridin, Thienyl, Morpholinyl und Piperidinyl.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Bevorzugt sind Verbindungen der allgemeinen Formel (I),
in welcher
- R¹: fiir Cyclopropyl, Cyclobutyl, Cyclopentyl, Cyclohexyl oder Cycloheptyl steht, oder
für einen Rest der Formel 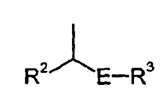 steht,
worin
- R²: geradkettiges oder verzweigtes Alkyl mit bis zu 8 Kohlenstoffatomen bedeutet, das gegebenenfalls durch Hydroxy substituiert ist,
und
- E: einen Rest der Formel -CH₂-T- bedeutet,
worin
- T: eine geradkettige oder verzweigte Alkylenkette mit bis zu 8 Kohlenstoffatomen bedeutet,
- R³: Wasserstoff oder Phenyl bedeutet, das gegebenenfalls bis zu 2-fach gleich oder verschieden durch Fluor, Chlor, Brom, Hydroxy, Nitro, Trifluormethyl oder durch geradkettiges oder verzweigtes Alkyl oder Alkoxy mit jeweils bis zu 5 Kohlenstoffatomen substituiert ist,
- A und D: für Wasserstoff stehen,
oder
- A: für Wasserstoff
und
- D: für Hydroxy steht,
oder
- A und D: gemeinsam für einen Rest der Formel =O stehen,
- L: für einen Rest der Formel 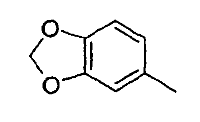 steht, oder
für Phenyl, Naphthyl, Pyridyl, Thienyl, Indolyl oder Furyl steht, die gegebenenfalls bis zu 3-fach gleich oder verschieden durch Substituenten ausgewählt aus der Gruppe Fluor, Chlor, Brom, Trifluormethyl, Hydroxy, Nitro, Carboxy, geradkettiges oder verzweigtes Alkyl, Alkoxy und Alkoxycarbonyl mit jeweils bis zu 5 Kohlenstoffatomen und/oder durch einen Rest der Formel -(V)ₐNR⁴R⁵ substituiert sind,
worin
- a: eine Zahl 0 oder 1 bedeutet,
- V: einen Rest der Formel -CO oder -SO₂ bedeutet,
- R⁴ und R⁵: gleich oder verschieden sind und Wasserstoff oder geradkettiges oder verzweigtes Acyl oder Alkoxycarbonyl mit jeweils bis zu 4 Kohlenstoffatomen bedeuten, oder
geradkettiges oder verzweigtes Alkyl mit jeweils bis zu 5 Kohlenstoffatomen bedeuten, das gegebenenfalls durch Hydroxy, Amino oder durch geradkettiges oder verzweigtes Alkyl- oder Dialkylamino mit jeweils bis zu 5 Kohlenstoffatomen substituiert ist,
oder
- R⁴ und R⁵: gemeinsam mit dem Stickstoffatom einen Morpholinyl-, Piperidinyl- oder Piperazinylring bilden, der gegebenenfalls über ein Stickstoffatom durch geradkettiges oder verzweigtes Alkyl mit bis zu 3 Kohlenstoffaotmen substituiert ist, und die gegebenenfalls durch geradkettiges oder verzweigtes Alkoxycarbonyl mit bis zu 4 Kohlenstoffatomen substituiert sind,
und/oder die unter L aufgeführten Ringsysteme gegebenenfalls durch Naphthyl, Phenyl, Pyridyl, Indolyl, Thienyl und Furyl substituiert sind, die gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(V')_{b}NR⁷R⁸ substituiert sind,
worin
- b: die oben angegebene Bedeutung von a hat und mit dieser gleich oder verschieden ist,
- V': die oben angegebene Bedeutung von V hat und mit dieser gleich oder verschieden ist,
- R⁷ und R⁸: die oben angegebene Bedeutung von R⁴ und R⁵ haben,
und deren Tautomere und Salze.

Besonders bevorzugt sind Verbindungen der allgemeinen Formel (I),
in welcher
- R¹: für Cyclopropyl, Cyclobutyl, Cyclopentyl, Cyclohexyl oder Cycloheptyl steht, oder
für einen Rest der Formel 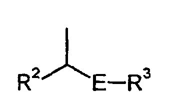 steht,
worin
- R²: geradkettiges oder verzweigtes Alkyl mit bis zu 7 Kohlenstoffatomen bedeutet, das gegebenenfalls durch Hydroxy substituiert ist,
und
- E: einen Rest der Formel -CH₂-T- bedeutet,
worin
- T: eine geradkettige oder verzweigte Alkylenkette mit bis zu 7 Kohlenstoffatomen bedeutet,
- R³: Wasserstoff oder Phenyl bedeutet, das gegebenenfalls bis zu 2-fach gleich oder verschieden durch Fluor, Chlor, Brom, Hydroxy, Nitro, Trifluormethyl oder durch geradkettiges oder verzweigtes Alkyl oder Alkoxy mit jeweils bis zu 4 Kohlenstoffatomen substituiert ist,
- A und D: für Wasserstoff stehen,
oder
- A: für Wasserstoff
und
- D: für Hydroxy steht,
oder
- A und D: gemeinsam für einen Rest der Formel =O stehen,
- L: für einen Rest der Formel 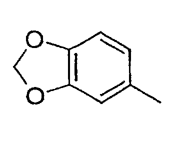 steht, oder
für Phenyl oder Pyridyl, steht, die gegebenenfalls bis zu 3-fach gleich oder verschieden durch Substituenten ausgewählt aus der Gruppe Fluor, Chlor, Brom, Trifluormethyl, Hydroxy, Nitro, Carboxy, geradkettiges oder verzweigtes Alkyl, Alkoxy und Alkoxycarbonyl mit jeweils bis zu 4 Kohlenstoffatomen und/oder durch einen Rest der Formel -(V)ₐNR⁴R⁵ substituiert sind,
worin
- a: eine Zahl 0 oder 1 bedeutet,
- V: einen Rest der Formel -CO oder -SO₂ bedeutet,
- R⁴ und R⁵: gleich oder verschieden sind und Wasserstoff oder geradkettiges oder verzweigtes Acyl oder Alkoxycarbonyl mit jeweils bis zu 3 Kohlenstoffatomen bedeuten, oder
geradkettiges oder verzweigtes Alkyl mit jeweils bis zu 4 Kohlenstoffatomen bedeuten, das gegebenenfalls durch Hydroxy, Amino oder durch geradkettiges oder verzweigtes Alkyl- oder Dialkylamino mit jeweils bis zu 3 Kohlenstoffatomen substituiert ist,
oder
- R⁴ und R⁵: gemeinsam mit dem Stickstoffatom einen Morpholinyl-, Piperidinyl- oder Piperazinylring bilden, der gegebenenfalls über ein Stickstoffatom durch geradkettiges oder verzweigtes Alkyl mit bis zu 3 Kohlenstoffaotmen substituiert ist, und die gegebenenfalls durch geradkettiges oder verzweigtes Alkoxycarbonyl mit bis zu 4 Kohlenstoffatomen substituiert sind,
und/oder die unter L aufgeführten Ringsysteme gegebenenfalls durch Phenyl oder Pyridyl substituiert sind,
und deren Tautomere und Salze.

Außerdem wurde ein Verfahren zur Herstellung der erfindungsgemäßen Verbindungen der allgemeinen Formel (I) gefunden, dadurch gekennzeichnet, daß man
im Fall A, D = H, Verbindungen der allgemeinen Formel (II), 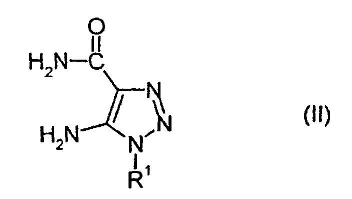 in welcher
- R¹: die oben angegebene Bedeutung hat,
mit Verbindungen der allgemeinen Formel (III)

L-CH₂-CO₂R⁹ (III)

in welcher
- L: die oben angegebene Bedeutung hat
und
- R⁹: für C₁-C₄-Alkyl steht,
in inerten Lösemitteln, gegebenenfalls in Anwesenheit einer Base, umsetzt
und gegebenenfalls die unter den Substituenten R¹, R³ und L angegebenen Substituenten durch Folgereaktionen wie Acylierung, Oxidation, Substitution und/oder Reduktionen einführt bzw. derivatisiert.

Das erfindungsgemäße Verfahren kann durch folgendes Formelschema beispielhaft erläutert werden:

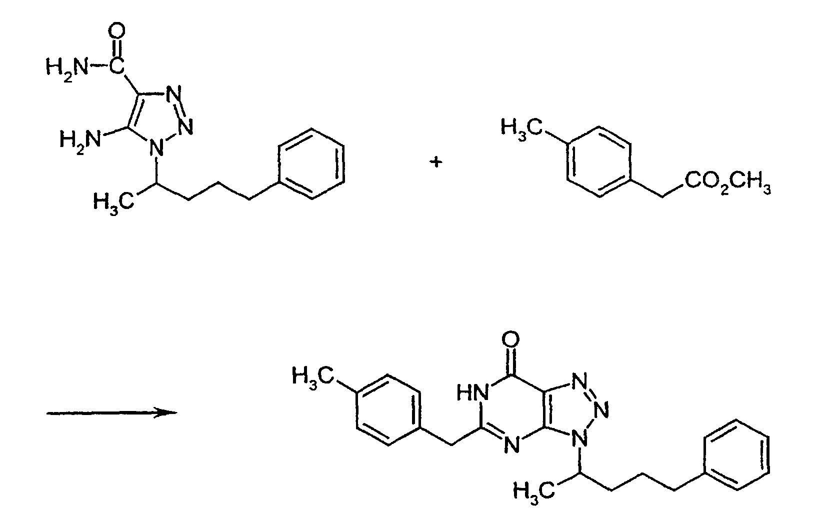

Als Lösemittel für das Verfahren eignen sich die üblichen organischen Lösemittel. Hierzu gehören bevorzugt Alkohole wie Methanol, Ethanol, Propanol, Isopropanol, Butanol oder t-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol, Acetonitril und Dimethylformamid verwendet. Ebenso ist es möglich, Gemische der genannten Lösemittel einzusetzen.

Als Basen eignen sich für das Verfahren die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Natriummethanolat und Nateriumethanolat.

Bei der Durchführung des Verfahrens wird die Base im allgemeinen in einer Menge von 2 bis 6 mol, bevorzugt von 3 bis 5 mol bezogen auf 1 mol der entsprechenen Amide, eingesetzt.

Das Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Umsetzung mit Alkylsulfonsäurechloriden erfolgt, ausgehend von den entsprechenden freien Hydroxyverbindungen, in einem der oben aufgeführten Lösemittel und einer der Basen, vorzugsweise mit Dichlormethan, Triethylamin oder Pyridin in einem Temperaturbereich von -20C bis +20°C, vorzugsweise 0°C und Normaldruck.

Die Einführung des Azidrestes erfolgt im allgemeinen durch Umsetzung der entsprechenden Alkylsulfonyloxy substituierten Verbindungen mit Natriumazid in einem der oben aufgeführten Lösemittel, vorzugsweise Dimethylformamid und Dimethylsulfonyl, in einem Temperaturbereich von 50°C bis +120°C, vorzugsweise 50°C und Normaldruck.

Die enantiomerenreinen Verbindungen sind nach üblichen Methoden, beispielsweise durch Chromatographie der racemischen Verbindungen der allgemeinen Formel (I) auf chiralen Phasen oder durch die Verwendung chiraler Ausgangsverbindungen zugänglich.

Die Verbindungen der allgemeinen Formel (II) sind teilweise bekannt und teilweise neu und können beispielsweise hergestellt werden, indem man Verbindungen der allgemeinen Formel (IV)

N₃-R¹ (IV)

in welcher
- R¹: die oben angegebene Bedeutung hat,
mit Verbindungen der Formel (V)

N≡C-CH₂-CO-NH₂ (V)

in inerten Lösemitteln in Anwesenheit einer Base umsetzt.

Als Lösemittel für das Verfahren eignen sich die üblichen organischen Lösemittel. Hierzu gehören bevorzugt Alkohole wie Methanol, Ethanol, Propanol, Isopropanol, Butanol oder t-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol, Acetonitril, Dimethylformamid und Dimethylsulfoxid verwendet. Ebenso ist es möglich, Gemische der genannten Lösemittel einzusetzen.

Als Basen eignen sich für das Verfahren die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Natriummethanolat und Natriumethanolat.

Das Verfahren wird im allgemeinen in einem Temperaturbereich von 0°C bis +180°C, bevorzugt von +20°C bis +150°C durchgerührt.

Das Verfahren wird im allgemeinen bei Normaldruck durchgeführt. Es ist aber auch möglich, bei Überdruck oder bei Unterdruck zu arbeiten (z.B. in einem Bereich von 0,5 bis 5 bar).

Die Verbindungen der allgemeinen Formeln (IV) und (V) sind an sich bekannt oder nach üblichen Methoden herstellbar.

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der c-GMP metabolisierenden Phosphodiesterasen (PDE I, PDE II und PDE V). Dies führt zu einem Anstieg von c-GMP. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren, eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Sie können daher in Arzneimitteln zur Behandlung von cardiovaskulären Erkrankungen wie beispielsweise zur Behandlung des Bluthochdrucks, neuronaler Hypertonie, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, Angina pectoris, peripheren Durchblutungsstörungen, Verhinderung von Restenosen nach Thrombolysetherapie, percutaner transluminaler Angioplastie (PTA), percutan transluminalen Koronarangioplastien (PTCA) und Bypass eingesetzt werden. Weiterhin können sie auch Bedeutung für cerebrovaskuläre Erkrankungen haben. Die relaxierende Wirkung auf glatte Muskulatur macht sie geeignet für die Behandlung von Erkrankungen des Urogenitalsystems wie Prostatahypertrophie, Inkontinenz sowie insbesondere zur Behandlung der erektilen Dysfunktion und der weiblichen sexuellen Dysfunktion.

### Aktivität der Phosphordiesterasen (PDE's)

Die c-GMP stimulierbare PDE II, die c-GMP hemmbare PDE III und die cAMP spezifische PDE IV wurden entweder aus Schweine- oder Rinderherzmyokard isoliert. Die Ca²⁺-Calmodulin stimulierbare PDE I wurde aus Schweineaorta, Schweinehirn oder bevorzugt aus Rinderaorta isoliert. Die c-GMP spezifische PDE V wurde aus Schweinedünndarm, Schweineaorta, humanen Blutplättchen und bevorzugt aus Rinderaorta gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990) und C. Lugman et al. Biochemical Pharmacology Vol. 35 1743-1751 (1986).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50 % des Substrates umgesetzt werden. Um die cGMP stimulierbare PDE II zu testen, wird als Substrat ³HcAMP verwendet und dem Ansatz 10⁻⁶ mol/l nicht markiertes cGMP zugesetzt. Um die Ca-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC getrennt und die Spaltprodukte "Online" mit einem Durchflußscintillationszähler quantitativ bestimmt. Es wird die Substanzkonzentration gemessen, bei der die Reaktionsgeschwindigkeit um 50 % vermindert ist. Zusätzlich wurde zur Testung der "Phosphodiesterase [³H] cAMP-SPA enzyme assay" und der "Phosphodiesterase [³H] cGMP-SPA enzyme assay" der Firma Amersham Life Science verwendet. Der Test wurde nach dem vom Hersteller angegebenen Versuchsprotokoll durchgeführt. Für die Aktivitätsbestimmung der PDE2 wurde der [³H] cAMP SPA assay verwendet, wobei dem Reaktionsansatz 10⁻⁶ M cGMP zur Aktivierung des Enzyms zugegeben wurde. Für die Messung der PDE1 wurden Calmodulin 10⁻⁷ M und CaCl₂ 1 µM zum Reaktionsansatz zugegeben. Die PDE5 wurde mit dem [³H] cGMP SPA assay gemessen.

| **Inhibition der Phosphodiesterasen in vitro** | | | |
|---|---|---|---|
| Bsp.-Nr. | PDE I IC₅₀ [nM] | PDE II IC₅₀ [nM] | PDE V IC₅₀ [nM] |
| 5 | 500 | 80 | 500 |
| 19 | 200 | 200 | >1000 |
| 23 | 100 | 100 | >1000 |
| 30 | 500 | 300 | =1000 |
| 31 | >1000 | 40 | >1000 |

Grundsätzlich führt die Inhibition einer oder mehrerer Phosphodiesterasen dieses Typs zu einer Erhöhung der cGMP-Konzentration. Dadurch sind die Verbindungen interessant für alle Therapien, in denen eine Erhöhung der cGMP-Konzentration als heilsam angenommen werden kann.

Die Untersuchung der cardiovaskulären Wirkungen wurden an SH-Ratten und Hunden durchgeführt. Die Substanzen wurden intravenös oder oral appliziert.

Die Untersuchung auf erektionsauslösendc Wirkung wurde am wachen Kaninchen durchgeführt [Naganuma H, Egashira T, Fuji J, Clinical and Experimental Pharmacology and Physiology 20, 177-183 (1993)]. Die Substanzen wurden intravenös, oral oder parenteral appliziert.

Die neuen Wirkstoffe sowie ihre physiologisch unbedenklichen Salze (z.B. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z.B. perlingual , buccal, intravenös, nasal, rektal oder inhalativ.

Trotzdem kann es gegebenenfalls erforderlich sein, von den unten genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der unten genannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Für die Anwendung beim Menschen werden bei oraler Administration Dosierungen von 0,001 bis 50 mg/kg vorzugsweise 0,01 mg/kg - 20 mg/kg sinnvollerweise verabreicht. Bei parenteraler Administration ist eine Dosierung von 0,001 mg/kg - 0,5 mg/kg sinnvoll.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

### Ausgangsverbindungen

### Beispiel I

### 5-Phenylpentan-2-ol

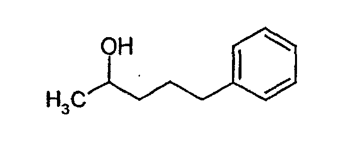

Zu einer Suspension von 5,2 g (213 mmol) Magnesium in 30 ml Diethylether gibt man auf einmal 3,6 ml (4,72 g, 23,7 mmol) 3-Phenylpropylbromid. Weitere 30 ml (39,3 g, 197 mmol) 3-Phenylpropylbromid werden so schnell zugetropft, daß ein konstanter Rückfluß erhalten bleibt. Nach beendeter Zugabe wird mit 100 ml Diethylether verdünnt und 30 min refluxiert. Es wird mit weiteren 80 ml Diethylether verdünnt und auf -78°C abbgekühlt. Eine 0°C kalte Lösung von 19 ml Acetaldehyd in 50 ml Ether wird zugetropft, die Reaktionsmischung 2 Stunden bei -78°C gerührt und auf Raumtemperatur aufgewärmt. 50 ml gesättigte NH₄Cl Lösung werden zugegeben; die organische Phase wird mit 1N HCl und gesättigter NaHCO₃ Lösung extrahiert und über Na₂SO₄ getrocknet. Nach Entfernen des Lösungsmittels erhält man 33,4 g (95 %) eines schwach gelben Öls.
R_{f}= 0,50 (Petrolether/Essigester = 7:3)

### Beispiel II

### Methansulfonsäure-1-methyl-4-phenylbutylester

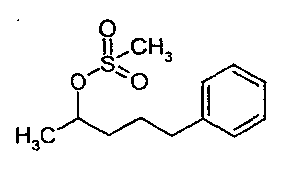

32,55 g 5-Phenylpentan-2-ol (198 mmol) werden in 500 ml Pyridin gelöst und bei Raumtemperatur mit 31 ml (45,88 g, 400 mmol) Mesylchlorid versetzt. Das Lösungsmittel wird nach 30 minütigem Rühren bei Raumtemperatur im Vakuum entfert und der Rückstand mit 200 ml CH₂Cl₂ aufgenommen. Es wird mit 1N HCl und gesättigter NaHCO₃ Lösung extrahiert, über Na₂SO₄ getrocknet und einrotiert. Man erhält 54,01 g braunes Öl, das noch MesCl enthält.
R_{f} = 0,27 (Cyclohexan/Essigester = 3:1)

### Beispiel III

### (4-Azidopentyl)-benzol

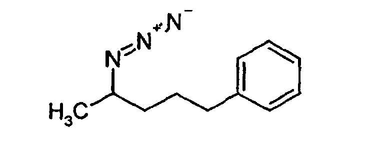

1,57 g Methansulfonsäure-1-methyl-4-phenylbutylester (6,5 mmol) werden in 30 ml DMSO gelöst, die Lösung wird mit 1,66 g NaN₃ (25,5 mmol) versetzt und 15 Stunden bei 50°C gerührt. Es wird mit 150 ml Ether und 100 ml gesättigter wäßriger NaHCO₃ Lösung verdünnt, die organische Phase wird zweimal mit Wasser gewaschen, über Na₂SO₄ getrocknet und das Lösungsmittel im Vakuum entfernt. Man erhält 997 mg (81 %) eines gelben Öls.
R_{f}= 0,61 (Cyclohexan/Essigester = 3:1)

### Beispiel IV

### 5-Amino-1-(1-methyl-4-phenyl-butyl)-1H-[1,2,3]triazol-4-carbonsäureamid

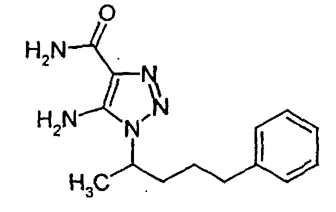

Eine Lösung von 997 mg (5,28 mmol) (4-Azidopentyl)-benzol und 672 mg (8 mmol) Cyanacetamid in 3 ml DMSO wird mit einer 50°C warmen Lösung von 127 mg (5,5 mmol) Natrium in 3 ml Ethanol versetzt. Die Reaktionsmischung wird 15 h bei Raumtemperatur gerührt und auf 25 ml Eiswasser gegossen. Es wird mit 1N HCl auf pH 2 eingestellt, der ausgefallenen Feststoff abgesaugt, mit Wasser gewaschen und im Vakuum getrocknet. Man erhält 1,14 g (79 %) eines schwach gelben Feststoffes.
Smp.: 117°C

### Beispiel V und Beispiel VI

### (4-Chlorsulfonyl-phenyl)-essigsäuremethylester (Beispiel V) und (3-Chlorsulfonylphenyl)-essigsäure methyl-ester (Beispiel VI)

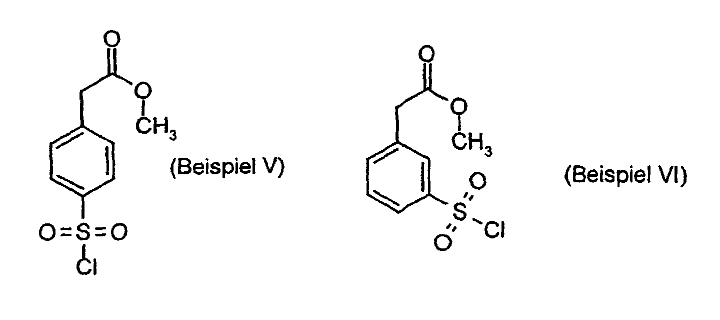

288 ml (2 mol) Phenylessigsäuremethylester werden langsam unter Eiskühlung zu 400 ml (6 mol) Chlorsulfonsäure getropft und die Reaktionsmischung wird 16 Stunden bei Raumtemperatur gerührt. Es wird auf 3 1 Eiswasser gegeben, dreimal mit Essigester extrahiert und die organische Phase über Natiumsulfat getrocknet. Chromatographisache Reinigung (Cyclohexan/Essigester = 3:1) ergibt 135,9 g (27,3 %) gelbes Öl.
R_{f} = 0,75 (Cyclohexan/ Essigester = 1:1).
Das entstandene Produkt enthält ca. 89 % des para- und 11 % des meta- Regioisomeren.

### Beispiel VII

### 3-Nonanol

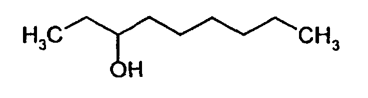

20,5 g (144 mmol) 3-Nonanon werden in 200 ml Methanol vorgelegt und nach portionsweiser Zugabe von 5,45 g (144 mmol) Natriumborhydrid bei Raumtemperatur über Nacht gerührt. Nach Entfernen des Lösungsmittel im Vakuum wird sauer gestellt und mit Dichlormethan extrahiert. Die organische Phase wird über Natriumsulfat getrocknet. Nach Entfernen des Lösungsmittels erhält man 20 g(96 %) farbloses Öl.
200 MHz-¹H-NMR (CDCl₃, ppm): 3,52, m, 1H; 1,48, m, 13H; 0,95, t, 3H; 0,89, t, 3H.

### Beispiel VIII

### Methansulfonsäure-1-ethyl-heptylester

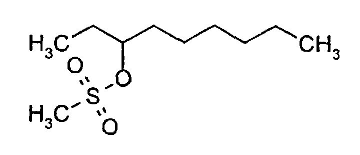

20 g(138,6 mmol) 3-Nonanol und 15,43 g Triethylamin werden in 150 ml Dichlormethan gelöst und tropfenweise mit 17,47 g (152,5 mmol) Mesylchlorid versetzt. Es wird 4 Stunden bei Raumtemperatur gerührt. Die organische Phase wird zweimal mit Wasser extrahiert, über Natriumsulfat getrocknet und das Lösungsmittel im Vakuum entfernt. Man erhält 30,7 g (95 %) einer gelben Flüssigkeit.
200 MHz-¹H-NMR (CDCl₃, ppm): 4,67, quin., 1H; 3,00, s, 3H; 1,71, m, 4H; 1,30, m, 8H; 0,99, t, 3H; 0,90, t, 3H.

### Beispiel IX

### 3-Azidononan

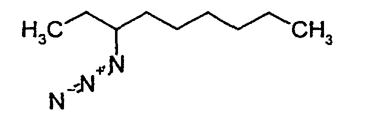

15,0 g (67,46 mmol) Methansulfonsäure-1-ethyl-heptylester werden in 300 ml DMSO gelöst und nach Zugabe von 17,54 g (269,8 mmol) Natriumazid 16 Stunden bei 50°C gerührt. Die Reaktionsmischung wird auf Eiswasser gegossen und dreimal mit Ether extrahiert. Nach Trocknen über Natriumsulfat erhält man 11,4 g (96 %) einer gelben Flüssigkeit.
200 MHz-¹H-NMR (CDCl₃, ppm): 3,18, quin., 1H; 1,40, m, 12H; 0,99, t, 3H; 0,90, t, 3H.

### Beispiel X

### 5-Amino-1-(1-methyl-heptyl)-1H-[1,2,3]triazol-4-carbonsäureamid

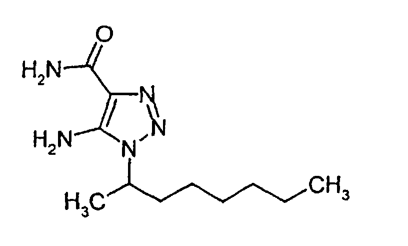

Eine Lösung von 23 g (135,9 mmol) 3-Azidononan und 17,14 g (203,8 mmol) Cyanacetamid in 80 ml DMSO wird mit einer Lösung von 3,25 g (141 mmol) Natrium in 80 ml Ethanol versetzt. Die Reaktionsmischung wird 15 h bei Raumtemperatur gerührt und auf 650 ml Eiswasser gegossen. Es wird mit 1N HCl auf pH 2 eingestellt, der ausgefallenen Feststoff abgesaugt, mit Wasser gewaschen und im Vakuum getrocknet. Man erhält 17,40 (50 %) eines schwach gelben Feststoffes.
200 MHz-¹H-NMR (CDCl₃, ppm): 6,81, s, breit, 1H; 5,50, s, breit, 1H; 5,01, s, breit, 2H; 3,91, quin., 1H; 1,92, m, 4H; 1,21, m, 8H; 0,86, m, 6H.

### Beispiel XI

### 5-Amino-1-[1-(1-hydroxy-ethyl)-4-phenyl-butyl]-1H-[1,2,3]triazol-4-carbonsäureamid

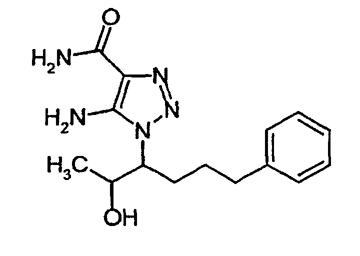

1,19 g(18,3 mmol) Natriumazid werden bei 0°C in einem Gemisch aus 3 ml Wasser und 5 ml Dichlormethan tropfenweise mit 1,046 g (mmol) Trifluormethansulfonsäureanhydrid versetzt und die resultierende Mischung 2 Stunden bei 0°C gerührt. Die Phasen werden getrennt, die wäßrige Phase wird zweimal mit Dichlormethan extrahiert und die vereinigten organischen Phasen mit gesättigter Natriumhydrogencarbonatlösung gewaschen. 386 mg (2 mmol) 3-Amino-6-phenyl-hexan-2-ol werden in 2 ml Methanol gelöst und nacheinander mit 10 mg Kupfersulfat und 10 mg Kaliumcarbonat versetzt. Dazu gibt man die Lösung des Triflylazids in Dichlormethan und füllt mit Dichlormethan auf ein Gesamtvolumen von 20 ml auf. Es wird 15 Stunden bei Raumtemperatur gerührt, mit verdünnter Ammoniaklösung und mit EDTA-Lösung extrahiert, über Natriumsulfat getrocknet und einrotiert. Man erhält 331 mg eines Öls, das in 1 ml DMSO gelöst und mit einer Lösung von 75 mg (3,5 mmol) Natrium in 1 ml Ethanol versetzt wird. Man gibt 197 mg (2,34 mmol) Cyanacetamid und nach 30 Minuten 2 ml Ethanol zu und rührt 15 Stunden bei Raumtemperatur. Man giesst auf 30 ml Eiswasser und extrahiert nach Zugabe von 3 ml 1N HCl dreimal mit Dichlormerthan und einmal mit Ethylacetat. Chromatographische Reinigung ergibt 72 mg (12 %) eines Schaums.
200 MHz-¹H-NMR (CDCl₃, ppm, 2 Diastereomere): 7,20, m, 5H; 5,69, s, breit, 1H; 5,48, s, breit, 2H; 5,36, s, breit, 1H; 4,24, m, 2H; 2,61, m, 2H; 2,31, m, 2H; 1,82, m, 2H; 1,55, m, 2H; 1,20, 2 d, 3H.

### Herstellungsbeispiele

### Beispiel 1

### 5-(4-Methyl-benzyl)-3-(1-methyl-4-phenyl-butyl)-3,6-dihydro-[1,2,3]triazolo[4,5-d]pyrimidin-7-on

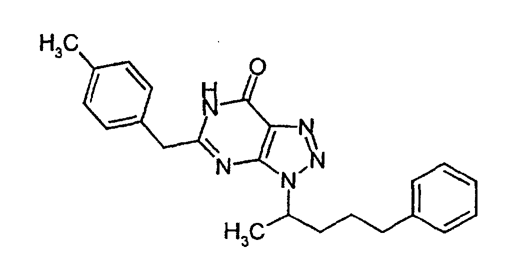

1,0 g (3,8 mmol) 5-Amino-1-(1-methyl-4-phenyl-butyl)-1*H*-[1,2,3]triazol-4-carbonsäureamid werden in einer Lösung von 439 mg Natrium (19 mmol) in 38 ml Ethanol gelöst. Nach Zugabe von 2,22 g (14,6 mmol) 4-Methylphenylessigsäuremethylester wird über Nacht refluxiert. Das Lösungsmittel wird im Vakuum entfernt, der Rückstand mit CH₂Cl₂ aufgenommen, die organische Phase zweimal mit 1N HCI extrahiert, über Na₂SO₄ getrocknet und das Lösungsmittel im Vakuum entfernt. Zweimalige chromatographische Reinigung (Cyclohexan/Essigester = 3:1) ergibt 853 mg (57 %) Feststoff.
Smp.: 130°C.

### Beispiel 2

### 3-(1-methyl-4-phenyl-butyl)-5-pyridin-4-ylmethyl-3,6-dihydro-[1,2,3]triazolo[4,5-d]pyrimidin-7-on

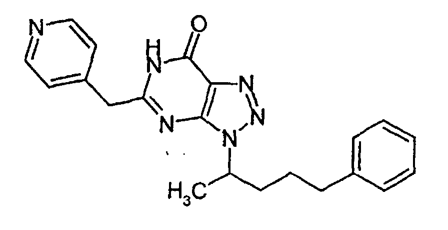

In Analogie zur Vorschrift des Beispiels 1 wird die Titelverbindung ausgehend von 1,0 g (3,8 mmol) 5-Amino-1-(1-methyl-4-phenyl-butyl)-1*H*-[1,2,3]triazol-4-carbonsäureamid und 2,21 g (14,6 mmol) 4-Pyridylessigsäuremethylester hergestellt.
Ausbeute: 266 mg (19 %) eines Schaums
R_{f}= 0,05 (Cyclohexan/Essigester = 1:1)

### Beispiel 3

### 5-(4-Bromo-benzyl)-3-(1-methyl-4-phenyl-butyl)-3,6-dihydro-[1,2,3]triazolo[4,5-d]pyrimidin-7-on

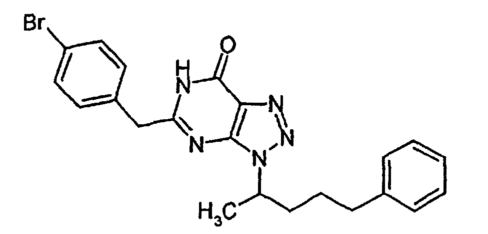

In Analogie zur Vorschrift des Beispiels 1 wird die Titelverbindung ausgehend von 1,0 g (3,8 mmol) 5-Amino-1-(1-methyl-4-phenyl-butyl)-1H-[1,2,3]triazol-4-carbonsäureamid und 2,61 g (11,4 mmol) 4-Bromphenylessigsäuremethylester hergestellt.
Ausbeute: 1,11 g (64 %) eines gelben Schaums
R_{f} = 0,59 (Cyclohexan/Essigester = 1:2)

### Beispiel 4

### 5-Benzyl-3-(1-methyl-4-phenyl-butyl)-3,6-dihydro-[1,2,3]triazolo[4,5-d]pyrimidin-7-on

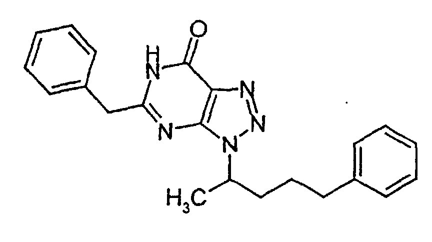

In Analogie zur Vorschrift des Beispiels 1 wird die Titelverbindung ausgehend von 566 mg (2 mmol) 5-Amino-1-(1-methyl-4-phenyl-butyl)-1H-[1,2,3]triazol-4-carbonsäureamid und 1,16 g (7,7 mmol) Phenylessigsäuremethylester hergestellt.
Ausbeute: 560 mg (75 %) Feststoff
Smp.: 112°C

### Beispiel 5

### 5-(3,4-Dimethoxy-benzyl)-3-(1-methyl-4-phenyl-butyl)-3,6-dihydro-[1,2,3]triazolo-[4,5-d]pyrimidin-7-on

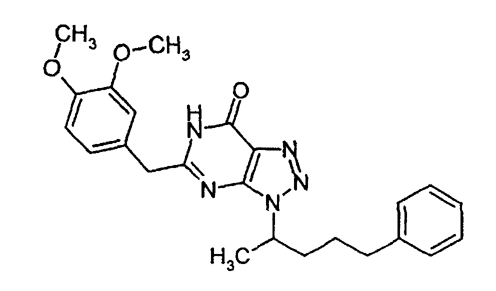

1,0g (3,8 mmol) 5-Amino-1-(1-methyl-4-phenyl-butyl)-1H-[1,2,3]triazol-4-carbonsäureamid, 2,13 g (19 mmol) KOtBu und 2,09 g (9,94 mmol) 3,4-Dimethoxyphenylessigsäuremethylester werden in 40 ml Ethanol gelöst und 15h refluxiert. Das Lösungsmittel wird im Vakuum entfernt, der Rückstand in CH₂Cl₂ aufgenommen und die organische Phase mit 2N HCl extrahiert. Nach Trocknen über Na₂SO₄ wird das Lösungsmittel im Vakuum enntfernt. Chromatographische Reinigung (Cyclohexan/Essigester = 1:1) ergibt 916 mg (56 %) eines Schaumes.
R_{f}= 0,49 (Cyclohexan/Essigester = 2:1)

### Beispiel 6

### 5-(4-Chlor-benzyl)-3-(1-methyl-4-phenyl-butyl)-3,6-dihydro-[1,2,3]triazolo[4,5-d]pyrimidin-7-on

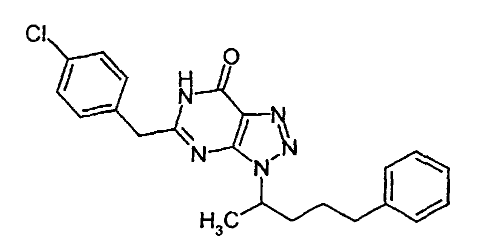

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 1,83 g (10,7 mmol) 4-Chlorphenylessigsäuremethylester hergestellt.
Ausbeute: 921 mg(59 %) weißen Schaum
R_{f}= 0,58 (Cyclohexan/Essigester = 4:1)

### Beispiel 7

### 5-(4-Methoxy-benzyl)-3-(1-methyl-4-phenyl-butyl)-3,6-dihydro-[1,2,3]triazolo[4,5-d]pyrimidin-7-on

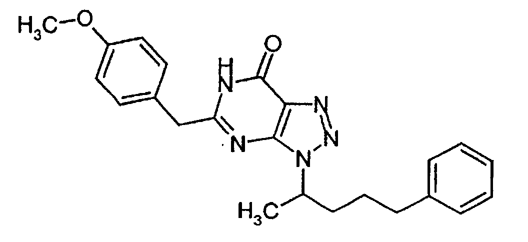

In Analogie zur Vorschrift des Beispiels 5 wird die Titelverbidnung ausgehend von 1,0 g (3,8 mmol) 5-Amino-1-(1-methyl-4-phenyl-butyl)-1H-[1,2,3]triazol-4-carbonsäureamid und 2,05 g (11,4 mmol) 4-Methoxyphenylessigsäuremethylester hergestellt.
Ausbeute: 850 mg (55 %) gelben Schaum
R_{f}= 0,49 (Cyclohexan/Essigester= 1:1)

### Beispiel 8

### 5-(3,4-Dichlor-benzyl)-3-(1-methyl-4-phenyl-butyl)-3,6-dihydro-[1,2,3]triazolo[4,5-d]pyrimidin-7-on

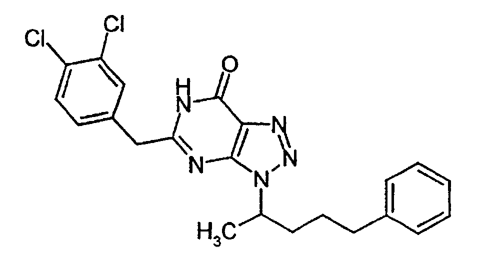

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 2,5 g (11,4 mmol) 3,4-Dichlorphenylessigsäuremethylester hergestellt.
Ausbeute: 1,08 g (64 %) weißen Schaum
R_{f}= 0,48 (Cyclohexan/Essigester = 4:1)

### Beispiel 9

### 5-Biphenyl-4-ylmethyl-3-(1-methyl-4-phenyl-butyl)-3,6-dihydro-[1,2,3]triazolo[4,5-d]pyrimidin-7-on

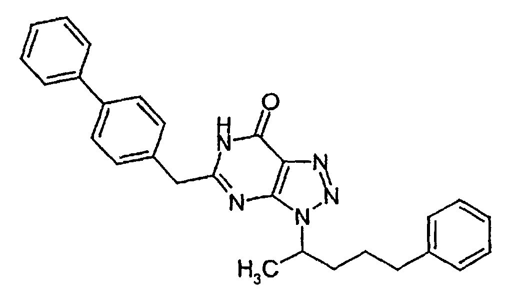

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 2,58 g (11,4 mmol) Biphenylessigsäuremethylester hergestellt.
Ausbeute: 1,03 g (64 %) weißen Schaum
R_{f}= 0,68 (Cyclohexan/Essigester = 4:1)

### Beispiel 10

### 5-(4-Amino-benzyl)-3-(1-methyl-4-phenyl-butyl)-3,6-dihydro-[1,2,3]triazolo[4,5-d]pyrimidin-7-on

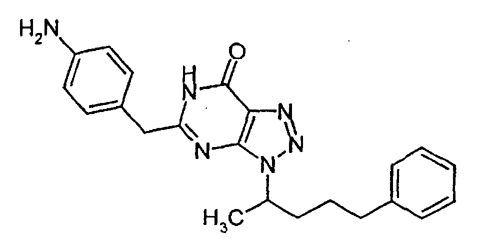

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 1,88 g (11,4 mmol) 4-Aminophenylessigsäure hergestellt.
Ausbeute: 600 mg (39 %) gelben Schaum
R_{f}=0,59 (Cyclohexan/Essigester=1:4)

### Beispiel 11

### 5-(Hydroxy-phenyl-methyl)-3-(1-methyl-4-phenyl-butyl)-3,6-dihydro-[1,2,3]triazolo[4,5-d]pyrimidin-7-on

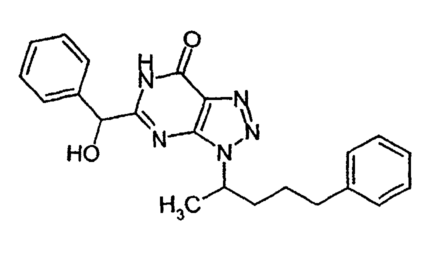

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 1,89 g (11,4 mmol) Mandelsäuremethylester hergestellt.
Ausbeute: 512 mg (34 %) weißen Schaum
R_{f}= 0,63 (Cyclohexan/Essigester = 1:4)

### Beispiel 12

### 5-(4-Fluor-benzyl)-3-(1-methyl-4-phenyl-butyl)-3,6-dihydro-[1,2,3]triazolo[4,5-d]pyrimidin-7-on

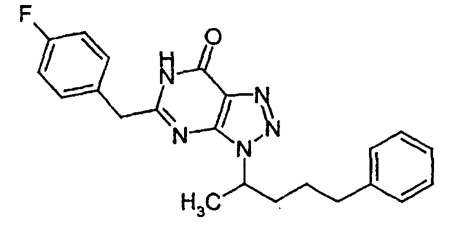

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 1,92 g (11,4 mmol) 4-Fluorphenylessigsäuremethylester hergestellt.
Ausbeute: 990 mg (67 %) gelben Schaum
R_{f} = 0,69 (CH/EE=1 :2)

### Beispiel 13

### 5-(4-Dimethylamino-benzyl)-3-(1-methyl-4-phenyl-butyl)-3,6-dihydro-[1,2,3]triazolo[4,5-d]pyrimidin-7-on

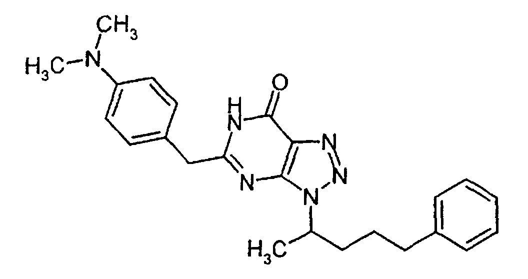

In Analogie zur Vorschrift des Beispiels 5 wird die Titelverbindung ausgehend von 1,0 g (3,8 mmol) 5-Amino-1-(1-methyl-4-phenyl-butyl)-1H-[1,2,3]triazol-4-carbonsäureamid und 1,77 g (9,15 mmol) 4-Dimethylaminophenylessigsäuremethylester hergestellt.
Ausbeute: 620 mg (41 %) gelben Schaum
R_{f}= 0,75 (Cyclohexan/Essigester = 1:4)

### Beispiel 14

### 5-Benzo[1,3]dioxol-5-ylmethyl-3-[1-methyl-4-phenyl-butyl]-3,6-dihydro-[1,2,3]triazolo[4,5-d]pyrimidin-7-on

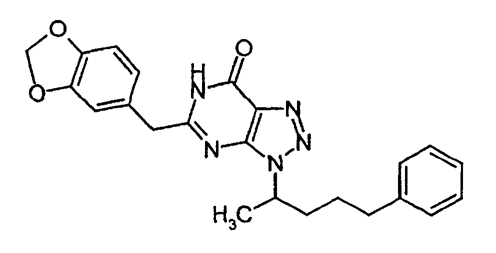

In Analogie zur Vorschrift des Beispiels 5 wird die Titelverbindung ausgehend von 18 mg (0,066 mmol) 5-Amino-1-(1-methyl-4-phenyl-butyl)-1H-[1,2,3]triazol-4-carbonsäureamid und 40,8 g (0,21 mmol) 3,4-Methylendioxyphenylessigsäuremethylester hergestellt.
Ausbeute: 22 mg (80 %) Feststoff
R_{f}= 0,31 (Dichlormethan/Methanol=95:5)

### Beispiel 15

*N*-{4-[3-(1-Methyl-4-phenyl-butyl)-7-oxo-6,7-dihydro-3*H*-[1,2,3]triazolo[4,5-*d*]pyrimidin-5-ylmethyl]-phenyl}-acetamid

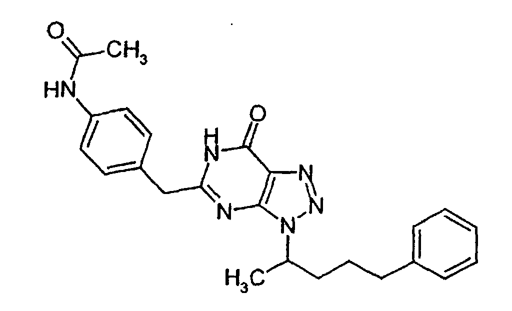

650 mg (1,62 mmol) 5-(4-Amino-benzyl)-3-(1-methyl-4-phenyl-butyl)-3,6-dihydro-[1,2,3]-triazolo-[4,5-*d*]pyrimidin-7-on werden in 10 ml Dichlormethan suspendiert und auf 0°C abgekühlt. Nacheinander werden 0,45 ml (3,24 mmol) Triethylamin, 0,23 ml (3,24 mmol) Acetylchlorid und eine Spatelspitze DMAP zugegeben und die Reaktionsmischung wird 3 Stunden bei Raumtemperatur gerührt. Es wird mit 100 ml Dichlormethan verdünnt und die organische Phase nach Waschen mit gesättigter Natriumhydrogencarbonatlösung, gesättigter Ammoniumchloridlösung und Wasser über Natriumsulfat getrocknet. Zweimalige chromatographische Reinigung ergibt 82 mg (11,5 %) eines weißen Schaumes.
R_{f}= 0,09 (Cyclohexan/Essigester = 1:2)

### Beispiel 16

### 5-Benzoyl-3-(1-methyl-4-phenyl-butyl)-3,6-dihydro-[1,2,3]triazolo[4,5-d]pyrimidin-7-on

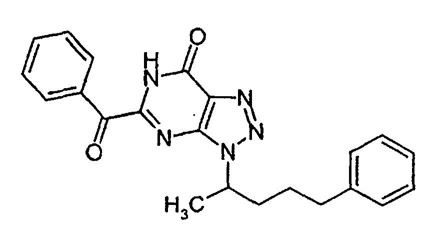

384 mg (0,988 mmol) 5-(Hydroxy-phenyl-methyl)-3-(1-methyl-4-phenyl-butyl)-3,6-dihydro-[1,2,3]triazolo[4,5-*d*]pyrimidin-7-on werden in einer Mischung aus 3 ml DMSO und 10 ml Dichlormethan gelöst und die Lösung auf 0°C abgekühlt. Nach Zugabe von 1,37 ml (9,88 mmol) Triethylamin und 692 mg (4,35 mmol) Pyridin-SO₃ Komplex wird die Reaktionsmischung 15 Stunden bei Raumtemperatur gerührt. Zur Aufarbeitung wird mit 100 ml Dichlormethan verdünnt und die organische Phase nach Waschen mit 1N HCl und gesättigter Natriumhydrogencarbonatlösung über Natriumsulfat getrocknet. Nach Entfernen des Lösungsmittels ergibt die chromatographische Reinigung 185 mg (48 %) eines Feststoffs, Smp.: 105°C

### Beispiel 17 und Beispiel 18

### 3-(1-Methyl-4-phenyl-butyl)-5-[4-(morpholin-4-sulfonyl)-benzyl]-3,6-dihydro-[1,2,3]triazolo-[4,5-d]pyrimidin-7-on (Beispiel 17) und 3-(1-Methyl-4-phenyl-butyl)-5-[3-(morpholin-4-sulfonyl)-benzyl]-3,6-dihydro[1,2,3]triazolo[4,5-d]pyrimidin-7-on (Beispiel 18)

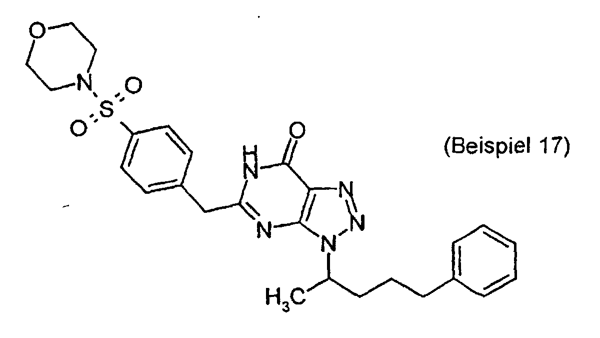

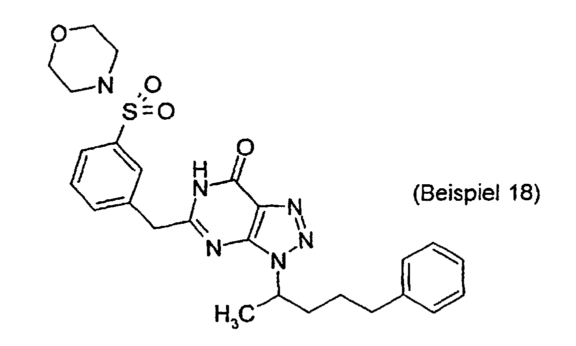

Zu einer Lösung von 1,54 g (13,7 mmol) KOtBu in 20 ml Ethanol gibt man 0,75 g (2,74 mmol) 5-Amino-1-(1-methyl-4-phenyl-butyl)-1H-[1,2,3]triazol-4-carbonsäureamid und eine Lösung von 1,92 g (6,13 mmol) 4-Morpholinosulfonylphenylessigsäuremethylester in 2 ml Ethanol. Die Reaktionsmischung wird 15 Stunden refluxiert und das Lösungsmittel im Vakuum entfernt. Der Rückstand wird mit 1N HCI neutral gestellt und in Dichlormethan aufgenommen. Die organische Phase wird mit Ammoniumchloridlösung und Wasser gewaschen und über Natriumsulfat getrocknet. Chromatographische Reinigung und Trennung der Regioisomere ergibt 334 mg (23 %) der Verbindung des Beispiels 17 und 186 mg (13 %) der Verbindung des Beispiels 18.

Beispiel 17: 200MHz-1H-NMR (CDCl₃, ppm): 12,8, s, breit, 1H; 7,68, s, breit, 4H; 7,19, m, 5H; 5,00, m, 1H; 4,22, s, 2H; 3,70, m, 4H; 2,95, m, 4H; 2,60, dt, 2H; 2,23, m, 1H; 2,00, m, 1H; 1,69, d, 3H; 1,56, m, 2H.

Beispiel 18: 200MHz-1H-NMR (CDCl₃, ppm): 12,81,s, breit, 1H; 7,90, s, 1H; 7,80, m, 2H; 7,50, m, 1H; 7,10, m, 5H; 4,98, m, 1H; 4,27, s, 2H; 3,68, m, 4H; 2,98, m, 2H; 2,60, m, 2H; 2,20, m, 1H; 1,98, m, 2H; 1,68, d, 3H; 1,48, m, 2H.

### Beispiel 19 und Beispiel 20

### N-Methyl-4-[3-(1-methyl-4-phenyl-butyl)-7-oxo-6,7-dihydro-3H-[1,2,3]-triazolo-[4,5-d]pyrimidin-5-ylmethyl]-benzolsulfonamid (Beispiel 19) und N-Methyl-3-[3-(1-methyl-4-phenyl-butyl)-7-oxo-6,7-dihydro-3H-[1,2,3]-triazolo-[4,5-d]pyrimidin-5-ylmethyl]-benzolsulfonamid (Beispiel 20)

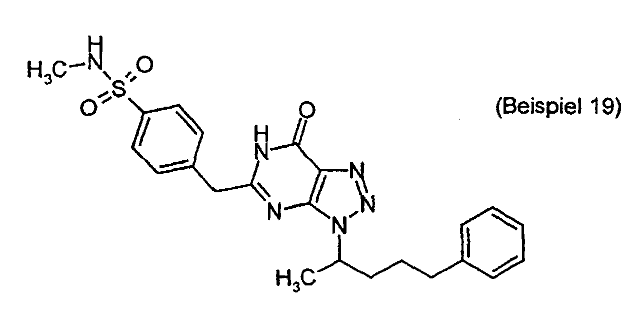

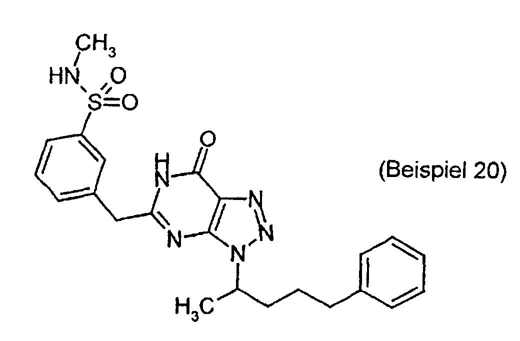

In Analogie zur Vorschrift der Beispiele 17 und 18 werden die Titelverbindungen ausgehend von 0,75 g 5-Amino-1-(1-methyl-4-phenyl-butyl)-1H-[1,2,3]triazol-4-carbonsäureamid und 1,58 g (6,14 mmol) 4-(N-methylsulfonylamid)-phenylessigsäuremethylester hergestellt.
(Beispiel 19) Ausbeute: 269 mg (21 %), Smp.:69°C und
(Beispiel 20) Ausbeute: 100 mg (7,8 %), Smp.:57°C

### Beispiel 21 und Beispiel 22

### N-(2-Dimethylamino-ethyl)-4-[3-(1-methyl-4-phenyl-butyl)-7-oxo-6,7-dihydro-3H-[1,2,3]triazolo[4,5-d]pyrimidin-5-ylmethyl]benzolsulfonamid (Beispiel 21) und N-(2-Dimethylamino-ethyl)-3-[3-(1-methyl-4-phenyl-butyl)-7-oxo-6,7-dihydro-3H-[1,2,3]triazolo [4,5-d]pyrimidin-5-ylmethyl]benzolsulfonamid (Beispiel 22)

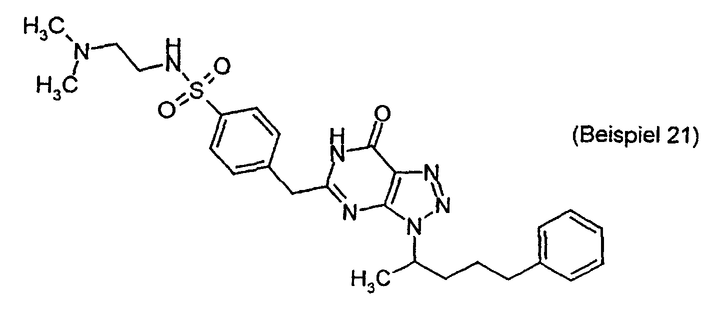

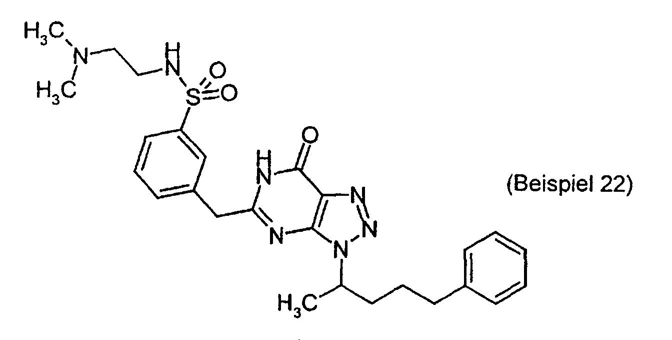

In Analogie zur Vorschrift der Beispiele 17 und 18 werden die Titelverbindungen ausgehend von 0,75 g 5-Amino-1-(1-methyl-4-phenyl-butyl)-1H-[1,2,3]triazol-4-carbonsäureamid und 1,89 g (6,01 mmol) 4-(N-2-Dimethylaminoethylsulfonylamid)-phenylessigsäuremethylester hergestellt.
(Beispiel 21): Ausbeute: 204 mg (14 %) und
(Beispiel 22): Ausbeute: 109 mg (7,6 %), R_{f}= 0,05 (Cyclohexan/Essigester = 1:1)

### Beispiel 23 und Beispiel 24

### N-(2-Hydroxyethyl)-4-[3-(1-methyl-4-phenyl-butyl)-7-oxo-6,7-dihydro-3H-[1,2,3]-triazolo[4,5-d]pyrimidin-5-ylmethyl]benzolsulfonamid (Beispiel 23) und N-(2-Hydroxyethyl)-3-[3-(1-methyl-4-phenyl-butyl)-7-oxo-6,7-dihydro-3H-[1,2,3]triazolo[4,5-d]pyrimidin-5-ylmethyl]benzolsulfonamid (Beispiel 24)

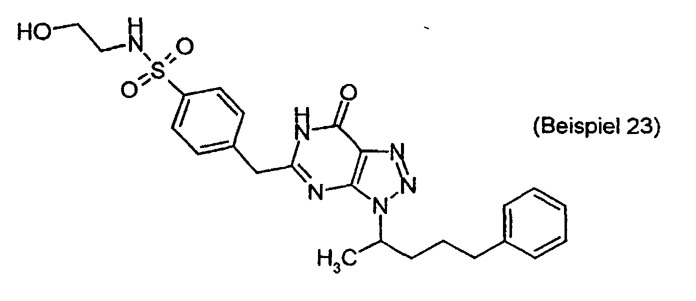

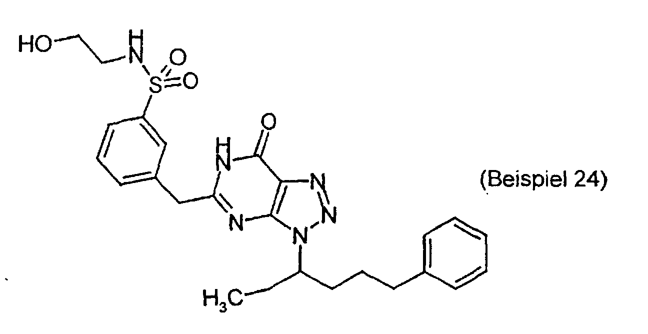

In Analogie zur Vorschrift der Beispiele 17 und 18 werden die Titelverbindungen ausgehend von 0,75 g 5-Amino-1-(1-methyl-4-phenyl-butyl)-lH-[1,2,3]triazol-4-carbonsäureamid und 1,97 g (6,85 mmol) 4-(N-2-Hydroxyethylsulfonylamid)-phenylessigsäuremethylester hergestellt.
(Beispiel 23) Ausbeute: 266 mg (20 %) R_{f}= 0,13 (Cyclohexan/Essigester = 1:1) und
(Beispiel 24): Ausbeute: 108 mg (8 %) R_{f}= 0,07 (Cyclo-hexan/Essigester = 1:1).

### Beispiel 25 und Beispiel 26

### 1-{4-[3-(1-methyl-4-phenyl-butyl)-7-oxo-6,7-dihydro-3H-[1,2,3]triazolo[4,5-d]pyrimidin-5-ylmethyl]benzolsulfonyl}-piperidincarbonsäureethylester (Beispiel 25) und 1-{3-[3-(1-methyl-4-phenyl-butyl)-7-oxo-6,7-dihydro-3H-[1,2,3]triazolo[4,5-d]pyrimidin-5-ylmethyl]benzolsulfonyl}-piperidincarbonsäureethylester (Beispiel 26)

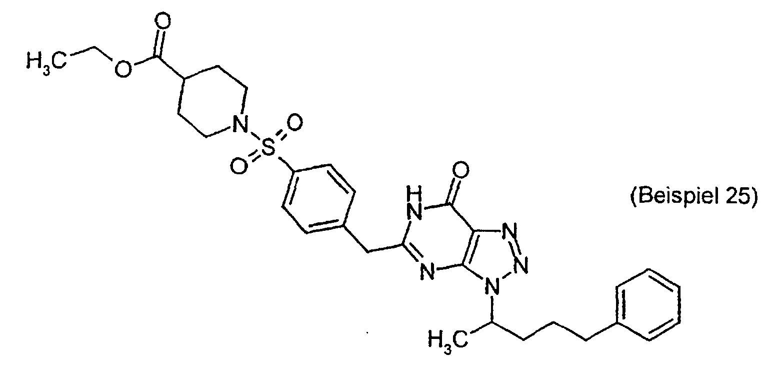

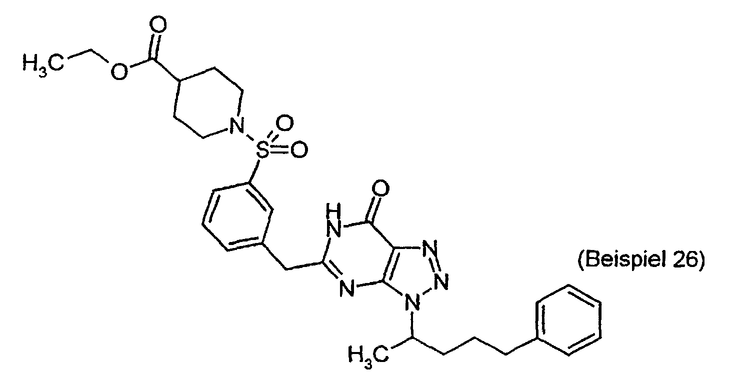

In Analogie zur Vorschrift der Beispiele 17 und 18 werden die Titelverbindungen ausgehend von 0,75 g 5-Amino-1-(1-methyl-4-phenyl-butyl)-1H-[1,2,3]triazol-4-carbonsäureamid und 2,40 g (5,85 mmol) N-(4-sulfonylphenylessigsäure-ethylester)-piperidincarbonsäuretert-butylester hergestellt.
(Beispiel 25) Ausbeute: 111 mg (7 %)
200 MHz-1H-NMR (CDCl₃, ppm): 12,63, s, breit, 1H; 7,62, AB, 4H; 7,20, m, 5H; 5,00, m, 1H; 4,21, s, 2H; 4,10, q, 2H; 3,60, m, 2H; 2,60,m, 2H; 2,48, m, 2H; 2,22, m, 2H; 1,90, m, 5H; 1,68, d, 3H; 1,60, m, 2H; 1,20, t, 2H
und
(Beispiel 26) Ausbeute: 43 mg (3 %)
200 MHz-1H-NMR (CDCl₃, ppm): 11,97, s, breit, 1H; 7,99, s, 1H; 7,67, m, 2H; 7,48, m, 1H; 7,18, m, 5H; 4,98, m, 1H; 4,22, s, 2H; 4,10, quart., 2H; 3,61, m, 2H; 2,55, m, 4H; 2,20, m, 2H; 1,90, m, 5H; 1,71, t, 3H; 1,68, d, 3H; 1,55, m, 2H.

### Beispiel 27

### 3-(1-methyl-4-phenyl-butyl)-5-[4-(4-methyl-piperazin-1-sulfonyl)-benzyl]-3,6-dihydro-[1,2,3]triazolo[4,5-d]pyrimidin-7-on

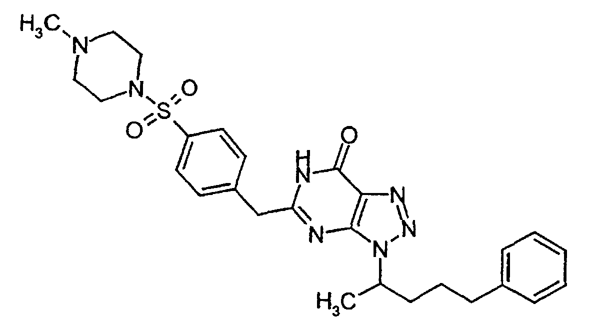

In Analogie zur Vorschrift der Beispiele 17 und 18 wird die Titelverbindung ausgehend von 0,75g 5-Amino-1-(1-methyl-4-phenyl-butyl)-1H-[1,2,3]triazol-4-carbonsäureamid und 1,97g (6,85 mmol) 4-(N-2-Hydroxyethylsulfonylamid)-phenylessigsäuremethylester hergestellt.
Ausbeute: 278 mg (19 %), R_{f}= 0,18 (Cyclohexan/Essigester = 1:1).

### Beispiel 28

### 5-Benzo[1,3]dioxol-5-ylmethyl-3-[1-ethyl-heptyl]-3,6-dihydro-[1,2,3]triazolo[4,5-d]pyrimidin-7-on

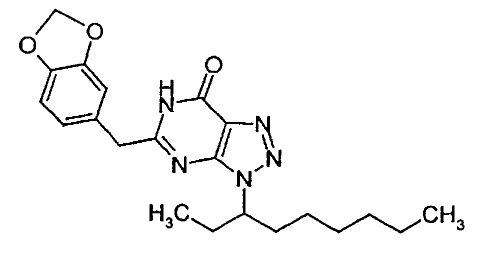

18 mg (0,071 mmol) 5-Amino-1-(1-methyl-heptyl)-1*H*-[1,2,3]triazol-4-carbonsäureamid und 40,8 mg (0,21 mmol) 3,4-Methylendioxyphenylessigsäuremethylester werden in 1,06 ml einer 0,5 M ethanolischen Kaliumtertbutylatlösung 6 Stunden am Rückfluß erhitzt. Es wird mit Dichlormethan und gesättigter Natriumhydrogencarbonatlösung verdünnt. Chromatographische Reinigung ergibt 23 mg (81 %) eines Feststoffs,
R_{f} = 0,42 (Dichlormethan/ Methanol = 95:5).

### Beispiel 29

### 5-(4-Brom-benzyl)-3-[1-(1-hydroxy-ethyl)-4-phenyl-butyl]-3,6-dihydro-[1,2,3]triazolo[4,5-d]pyrimidin-7-on

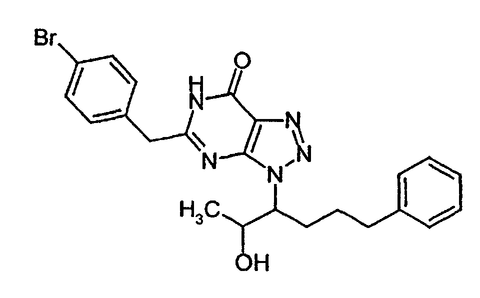

10 mg (0,024 mmol) 5-Amino-1-[1-(1-hydroxy-ethyl)-4-phenyl-butyl]-1*H*-[1,2,3]triazol-4-carbonsäureamid, 20 mg Kaliumtertbutylat (0,166 mmol) und 20 mg (0,107 mmol) 4-Brom-phenylessigsäuremethylester werden in 0,3 ml Ethanol 15 Stunden am Rückfluß erhitzt. Es wird mit Dichlormethan und Natriumhydrogencarbonatlösung verdünnt. Chromatographische Reinigung ergibt 8 mg (66 %) eines Feststoffs,
R_{f}= 0,46 (Dichlormethan/Methanol = 15:1)

### Beispiel 30

### 5-Benzo[1,3]dioxol-5-ylmetyl-3-[1-(1-hydroxy-ethyl)-4-phenyl-butyl]-3,6-dihydro-[1,2,3]triazolo[4,5-d]pyrimidin-7-on

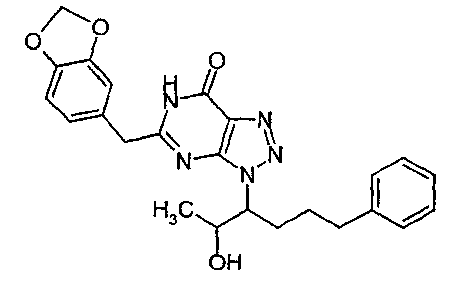

In Analogie zur Vorschrift des Beispiels 29 wird die Titelverbindung ausgehend von 10 mg (0,024 mmol) 5-Amino-1-[1-(1-hydroxy-ethyl)-4-phenyl-butyl]-1*H*-[1,2,3]triazol-4-carbon-säureamid und 20 mg (0,107 mmol) 3,4-Methylendioxyphenylessigsäuremethylester hergestellt.
Ausbeute: 7 mg (63 %)
R_{f}= 0,46 (Dichlormethan/Methanol = 15:1)

### Beispiel 31

### 5-(3,4-Dimethoxy-benzyl)-3-[1-(1-hydroxy-ethyl)-4-phenyl-butyl]-3,6-dihydro-[1,2,3]triazolo[4,5-d]pyrimidin-7-on

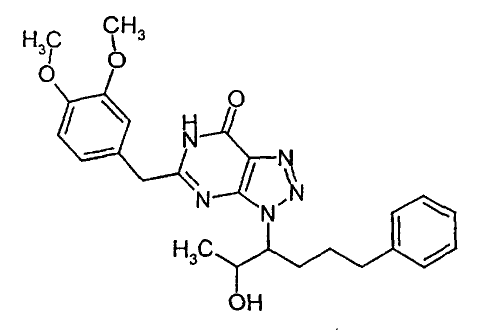

In Analogie zur Vorschrift des Beispiels 29 wird die Titelverbindung ausgehend von 10 mg (0,024 mmol) 5-Amino-1-[1-(1-hydroxy-ethyl)-4-phenyl-butyl]-1*H*-[1,2,3]triazol-4-carbon-säureamid und 20 mg (0,107 mmol) 3,4-Dimethoxyphenylessigsäuremethylester hergestellt.
Ausbeute: 7 mg (63 %)
R_{f}= 0,46 (Dichlormethan/Methanol = 15:1)

### Beispiel 32

### 5-(3,4,5-Trimethoxy-benzyl)-3-[1-(1-hydroxy-ethyl)-4-phenyl-butyl]-3,6-dihydro-[1,2,3]triazolo[4,5-d]pyrimidin-7-on

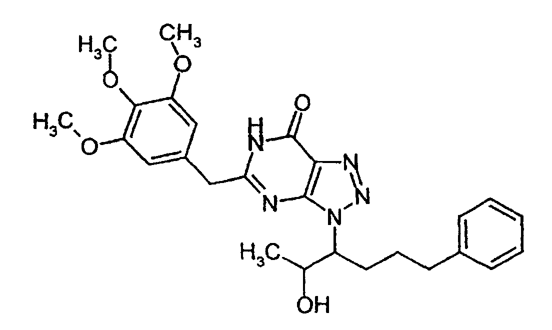

In Analogie zur Vorschrift des Beispiels XI wird die Titelverbindung ausgehend von 10 mg (0,024 mmol) 5-Amino-1-[1-(1-hydroxy-ethyl)-4-phenyl-butyl]-1*H*-[1,2,3]triazol-4-carbon-säureamid und 20 mg (0,107 mmol) 3,4,5-Trimethoxyphenylessigsäuremethylester hergestellt.
Ausbeute: 7 mg (63 %)
R_{f}= 0,44 (Dichlormethan/Methanol = 15:1)

### Beispiel 33

### 3-[1-(1-Hydroxy-ethyl)-4-phenyl-butyl]-5-[4-(morpholin-4-sulfonyl)-benzyl]-3,6-dihydro-[1,2,3]triazolo[4,5-d]pyrimidin-7-on

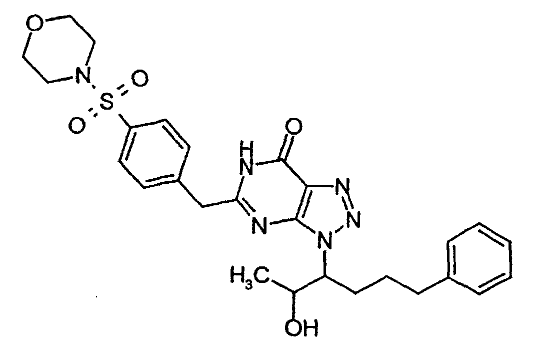

In Analogie zur Vorschrift des Beispiels 29 wird die Titelverbindung ausgehend von 10 mg (0,024 mmol) 5-Amino-1-[1-(1-hydroxy-ethyl)-4-phenyl-butyl]-1*H*-[1,2,3]triazol-4-carbon-säureamid und 30 mg (0,107 mmol) 4-Morpholinosulfonylphenylessigsäuremethylester hergestellt.
Ausbeute: 10 mg (75 %)
R_{f}= 0,37 (Dichlormethan/Methanol = 15:1)

## Patentansprüche

1. Dihydro-[1,2,3]triazolo-[4,5-d]pyrimidin-7-one der allgemeinen Formel (I) 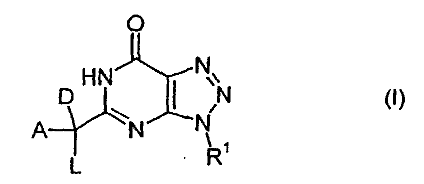 in welcher
R¹ für Cycloalkyl mit 3 bis 8 Kohlenstoffatomen steht, oder
für einen Rest der Formel 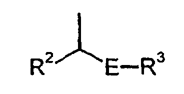 steht, worin
R² geradkettiges oder verzweigtes Alkyl mit bis zu 10 Kohlenstoffatomen bedeutet, das gegebenenfalls durch Hydroxy substituiert ist,
E einen Rest der Formel -CH₂-T bedeutet,
worin
T eine geradkettige oder verzweigte Alkylenkette mit bis zu 10 Kohlenstoffatomen bedeutet,
R³ Wasserstoff oder Aryl mit 6 bis 10 Kohlenstoffatomen bedeutet, das gegebenenfalls bis zu 3-fach gleich oder verschieden durch Halogen, Hydroxy, Nitro, Trifluormethyl oder durch geradkettiges oder verzweigtes Alkyl oder Alkoxy mit jeweils bis zu 6 Kohlenstoffatomen substituiert ist,
A und D für Wasserstoff stehen,
oder
A für Wasserstoff
und
D für Hydroxy steht,
oder
A und D gemeinsam für einen Rest der Formel =O stehen,
L für einen Rest der Formel 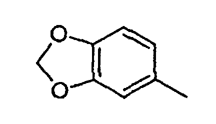 steht, oder
für Aryl mit 6 bis 10 Kohlenstoffatomen oder für einen 5- bis 7-gliedrigen aromatischen, gegebenenfalls benzokondensierten Heterocyclus mit bis zu 3 Heteroatomen aus der Reihe S, N und/oder O steht, wobei die oben unter L aufgeführten Ringsysteme bis zu 3-fach gleich oder verschieden substituiert sind durch einen oder mehrere der folgenden Substituenten: Halogen, Hydroxy, Nitro, Trifluormethyl, Carboxy, geradkettiges oder verzweigtes Alkyl, Alkoxy und Alkoxycarbonyl mit jeweils bis zu 6 Kohlenstoffatomen, einen Rest der Formel -(V)ₐ-NR⁴R⁵,
worin
a eine Zahl 0 oder 1 bedeutet,
V einen Rest der Formel -CO oder -SO₂ bedeutet,
R⁴ und R⁵ gleich oder verschieden sind und Wasserstoff oder geradkettiges oder verzweigtes Acyl oder Alkoxycarbonyl mit jeweils bis zu 6 Kohlenstoffatomen bedeuten, oder
geradkettiges oder verzweigtes Alkyl mit bis zu 6 Kohlenstoffatomen bedeuten, das gegebenenfalls durch Hydroxy, Amino oder durch geradkettiges oder verzweigtes Alkyl- oder Dialkylamino mit jeweils bis zu 6 Kohlenstoffatomen substituiert ist, oder
R⁴ und R⁵ gemeinsam mit dem Stickstoffatom einen 5- oder 6-gliedrigen, gesättigten Heterocyclus bilden, der gegebenenfalls ein weiteres Heteroatom aus der Reihe S, O oder einen Rest der Formel -NR⁶ enthalten kann und der gegebenenfalls durch geradkettiges oder verzweigtes Alkoxycarbonyl mit bis zu 6 Kohlenstoffatomen substituiert ist,
worin
R⁶ Wasserstoff oder geradkettiges oder verzweigtes Alkyl mit bis zu 4 Kohlenstoffatomen bedeutet,
und/oder die unter L aufgeführten Ringsysteme gegebenenfalls durch Aryl mit 6 bis 10 Kohlenstoffatomen und einen 5- bis 7-gliedrigen aromatischen, gegebenenfalls benzokondensierten Heterocyclus mit bis zu 3 Heteroatomen aus der Reihe S, N und/oder O substituiert sind, wobei diese Ringsysteme ihrerseits gegebenenfalls bis zu 2-fach gleich oder verschieden durch Substituenten aus der Reihe Halogen, Hydroxy, Nitro, Carboxyl, Trifluormethyl oder durch geradkettiges oder verzweigtes Alkyl, Alkoxy oder Alkoxycarbonyl mit jeweils bis zu 5 Kohlenstoffatomen oder durch eine Gruppe der Formel -(V')_{b} -NR⁷R⁸ substituiert sind,
worin
b die oben angegebene Bedeutung von a hat und mit dieser gleich oder verschieden ist,
R⁷ und R⁸ die oben angegebene Bedeutung von R⁴ und R⁵ haben und mit dieser gleich oder verschieden sind,
V' die oben angegebene Bedeutung von V hat und mit dieser gleich oder verschieden ist,
und deren Tautomere und Salze.

2. Dihydro-[1,2,3]triazolo-[4,5-d]pyrimidin-7-one nach Anspruch 1, **dadurch gekennzeichnet, daß**
R¹ für Cyclopropyl, Cyclobutyl, Cyclopentyl, Cyclohexyl oder Cycloheptyl steht, oder
für einen Rest der Formel 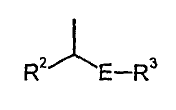 steht,
worin
R² geradkettiges oder verzweigtes Alkyl mit bis zu 8 Kohlenstoffatomen bedeutet, das gegebenenfalls durch Hydroxy substituiert ist,
und
E einen Rest der Formel -CH₂-T- bedeutet,
worin
T eine geradkettige oder verzweigte Alkylenkette mit bis zu 8 Kohlenstoffatomen bedeutet,
R³ Wasserstoff oder Phenyl bedeutet, das gegebenenfalls bis zu 2-fach gleich oder verschieden durch Fluor, Chlor, Brom, Hydroxy, Nitro, Trifluormethyl oder durch geradkettiges oder verzweigtes Alkyl oder Alkoxy mit jeweils bis zu 5 Kohlenstoffatomen substituiert ist,
A und D für Wasserstoff stehen,
oder
A für Wasserstoff
und
D für Hydroxy steht,
oder
A und D gemeinsam für einen Rest der Formel =O stehen,
L für einen Rest der Formel 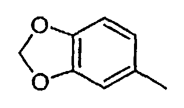 steht, oder
für Phenyl, Naphthyl, Pyridyl, Thienyl, Indolyl oder Furyl steht, die gegebenenfalls bis zu 3-fach gleich oder verschieden durch Substituenten ausgewählt aus der Gruppe Fluor, Chlor, Brom, Trifluormethyl, Hydroxy, Nitro, Carboxy, geradkettiges oder verzweigtes Alkyl, Alkoxy und Alkoxycarbonyl mit jeweils bis zu 5 Kohlenstoffatomen und/oder durch einen Rest der Formel -(V)ₐNR⁴R⁵ substituiert sind,
worin
a eine Zahl 0 oder 1 bedeutet,
V einen Rest der Formel -CO oder -SO₂ bedeutet,
R⁴ und R⁵ gleich oder verschieden sind und Wasserstoff oder geradkettiges oder verzweigtes Acyl oder Alkoxycarbonyl mit jeweils bis zu 4 Kohlenstoffatomen bedeuten, oder
geradkettiges oder verzweigtes Alkyl mit jeweils bis zu 5 Kohlenstoffatomen bedeuten, das gegebenenfalls durch Hydroxy, Amino oder durch geradkettiges oder verzweigtes Alkyl- oder Dialkylamino mit jeweils bis zu 5 Kohlenstoffatomen substituiert ist,
oder
R⁴ und R⁵ gemeinsam mit dem Stickstoffatom einen Morpholinyl-, Piperidinyl- oder Piperazinylring bilden, der gegebenenfalls über ein Stickstoffatom durch geradkettiges oder verzweigtes Alkyl mit bis zu 3 Kohlenstoffaotmen substituiert ist, und die gegebenenfalls durch geradkettiges oder verzweigtes Alkoxycarbonyl mit bis zu 4 Kohlenstoffatomen substituiert sind,
und/oder die unter L aufgeführten Ringsysteme gegebenenfalls durch Naphthyl, Phenyl, Pyridyl, Indolyl, Thienyl und Furyl substituiert sind, die gegebenenfalls durch Fluor, Chlor, Brom, Hydroxy, Nitro, Carboxyl, Trifluormethyl oder durch geradkettiges oder verzweigtes Alkyl, Alkoxy oder Alkoxycarbonyl mit jeweils bis zu 3 Kohlenstoffatomen oder durch eine Gruppe der Formel -(V')_{b}NR⁷R⁸ substituiert sind,
worin
b die oben angegebene Bedeutung von a hat und mit dieser gleich oder verschieden ist,
V' die oben angegebene Bedeutung von V hat und mit dieser gleich oder verschieden ist,
R⁷ und R⁸ die oben angegebene Bedeutung von R⁴ und R⁵ haben,
und deren Tautomere und Salze.

3. Dihydro-[1,2,3]triazolo-[4,5-d]pyrimidin-7-one nach Anspruch 1 oder 2, **dadurch gekennzeichnet, daß**
R¹ für Cyclopropyl, Cyclobutyl, Cyclopentyl, Cyclohexyl oder Cycloheptyl steht, oder
für einen Rest der Formel 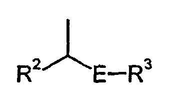 steht,
worin
R² geradkettiges oder verzweigtes Alkyl mit bis zu 7 Kohlenstoffatomen bedeutet, das gegebenenfalls durch Hydroxy substituiert ist,
und
E einen Rest der Formel -CH₂-T- bedeutet,
worin
T eine geradkettige oder verzweigte Alkylenkette mit bis zu 7 Kohlenstoffatomen bedeutet,
R³ Wasserstoff oder Phenyl bedeutet, das gegebenenfalls bis zu 2-fach gleich oder verschieden durch Fluor, Chlor, Brom, Hydroxy, Nitro, Trifluormethyl oder durch geradkettiges oder verzweigtes Alkyl oder Alkoxy mit jeweils bis zu 4 Kohlenstoffatomen substituiert ist,
A und D für Wasserstoff stehen,
oder
A für Wasserstoff
und
D für Hydroxy steht,
oder
A und D gemeinsam für einen Rest der Formel =O stehen,
L für einen Rest der Formel 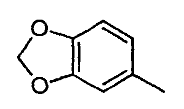 steht, oder
für Phenyl oder Pyridyl, steht, die gegebenenfalls bis zu 3-fach gleich oder verschieden durch Substituenten ausgewählt aus der Gruppe Fluor, Chlor, Brom, Trifluormethyl, Hydroxy, Nitro, Carboxy, geradkettiges oder verzweigtes Alkyl, Alkoxy und Alkoxycarbonyl mit jeweils bis zu 4 Kohlenstoffatomen und/oder durch einen Rest der Formel -(V)ₐNR⁴R⁵ substituiert sind,
worin
a eine Zahl 0 oder 1 bedeutet,
V einen Rest der Formel -CO oder -SO₂ bedeutet,
R⁴ und R⁵ gleich oder verschieden sind und Wasserstoff oder geradkettiges oder verzweigtes Acyl oder Alkoxycarbonyl mit jeweils bis zu 3 Kohlenstoffatomen bedeuten, oder
geradkettiges oder verzweigtes Alkyl mit jeweils bis zu 4 Kohlenstoffatomen bedeuten, das gegebenenfalls durch Hydroxy, Amino oder durch geradkettiges oder verzweigtes Alkyl- oder Dialkylamino mit jeweils bis zu 3 Kohlenstoffatomen substituiert ist,
oder
R⁴ und R⁵ gemeinsam mit dem Stickstoffatom einen Morpholinyl-, Piperidinyl- oder Piperazinylring bilden, der gegebenenfalls über ein Stickstoffatom durch geradkettiges oder verzweigtes Alkyl mit bis zu 3 Kohlenstoffaotmen substituiert ist, und die gegebenenfalls durch geradkettiges oder verzweigtes Alkoxycarbonyl mit bis zu 4 Kohlenstoffatomen substituiert sind,
und/oder die unter L aufgeführten Ringsysteme gegebenenfalls durch Phenyl oder Pyridyl substituiert sind,
und deren Tautomere und Salze.

4. Verfahren zur Herstellung der Dihydro-[1,2,3]triazolo-[4,5-d]pyrimidin-7-one nach Anspruch 1 bis 3, **dadurch gekennzeichnet, daß** man für den Fall, daß A und D für Wasserstoff stehen Verbindungen der allgemeinen Formel (II), 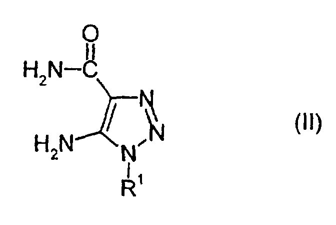 in welcher
R¹ die oben angegebene Bedeutung hat,
mit Verbindungen der allgemeinen Formel (III)
L-CH₂-CO₂R⁹ (III)
in welcher
L die oben angegebene Bedeutung hat
und
R⁹ für C₁-C₄-Alkyl steht,
in inerten Lösemitteln, gegebenenfalls in Anwesenheit einer Base, umsetzt
und gegebenenfalls die unter den Substituenten R¹, R³ und L angegebenen Substituenten durch Folgereaktionen wie Acylierung, Oxidation, Substitution und/oder Reduktionen einführt bzw. derivatisiert.

5. Arzneimittel enthaltend ein oder mehrere Dihydro-[1,2,3]triazolo-[4,5-d]-pyrimidin-7-one nach Anspruch 1 bis 3 in Kombination mit den üblichen Hilfs- und Trägerstoffen.

6. Verwendung von Dihydro-[1,2,3]triazolo-[4,5-d]pyrimidin-7-onen nach Anspruch 1 bis 3 in Kombination mit den üblichen Hilfs- und Trägerstoffen zur Herstellung von Arzneimitteln.

7. Verwendung von Dihydro-[1,2,3]triazolo-[4,5-d]pyrimidin-7-onen nach Anspruch 1 bis 3 in Kombination mit den üblichen Hilfs- und Trägerstoffen zur Herstellung von Arzneimitteln zur Behandlung von cardiovaskulären Erkrankungen wie beispielsweise zur Behandlung des Bluthochdrucks, neuronaler Hypertonie, stabiler und instabiler Angina, peripheren und kardialen Gefäßerkrankungen, von Arrhythmien, zur Behandlung von thromboembolischen Erkankungen und Ischämien wie Myokardinfakt, Hirnschlag, transistorischen und ischämischen Attacken, Angina pectoris, peripheren Durchblutungsstörungen, Verhinderung von Restenosen nach Thrombolysetherapie, percutaner transluminaler Angioplastie (PTA), percutan transluminalen Koronarangioplastien (PTCA) und Bypass sowie zur Behandlung von Erkrankungen des Urogenitalsystems wie Prostatahypertrophie, Inkontinenz sowie insbesondere zur Behandlung der erektilen Dysfunktion und der weiblichen sexuellen Dysfunktion.

## Claims

1. Dihydro-[1,2,3]triazolo-[4,5-d]pyrimidin-7-ones of the general formula (I) 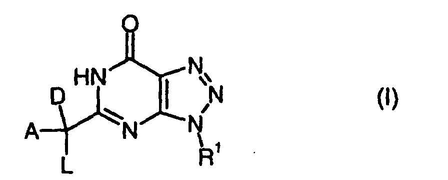 in which
R¹ represents cycloalkyl having 3 to 8 carbon atoms, or
represents a radical of the formula 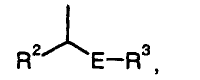 in which
R² xrepresents straight-chain or branched alkyl having up to 10 carbon atoms which is optionally substituted by hydroxyl,
E represents a radical of the formula -CH₂-T,
in which
T represents a straight-chain or branched alkylene chain having up to 10 carbon atoms,
R³ represents hydrogen or aryl having 6 to 10 carbon atoms which is optionally substituted up to 3 times by identical or different substitutents from the group consisting of halogen, hydroxyl, nitro, trifluoromethyl and straight-chain or branched alkyl or alkoxy having in each case up to 6 carbon atoms,
A and D represent hydrogen,
or
A represents hydrogen
and
D represents hydroxyl,
or
A and D together represent a radical of the formula =O,
L represents a radical of the formula 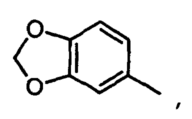 or
represents aryl having 6 to 10 carbon atoms or represents a 5- to 7-membered aromatic, optionally benzo-fused heterocycle having up to 3 heteroatoms from the group consisting of S, N and/or O, where the ring systems listed above under L are substituted up to 3 times by one or more identical or different of the following substituents: halogen, hydroxyl, nitro, trifluoromethyl, carboxyl, straight-chain or branched alkyl, alkoxy and alkoxycarbonyl having in each case up to 6 carbon atoms, a radical of the formula -(V)ₐ-NR⁴R⁵,
in which
a represents a number 0 or 1,
V represents a radical of the formula -CO or -SO₂,
R⁴ and R⁵ are identical or different and represent hydrogen or straight-chain or branched acyl or alkoxycarbonyl having in each case up to 6 carbon atoms, or
represent straight-chain or branched alkyl having up to 6 carbon atoms which is optionally substituted by hydroxyl, amino or by straight-chain or branched alkyl- or dialkylamino having in each case up to 6 carbon atoms, or
R⁴ and R⁵ together with the nitrogen atom form a 5-or 6-membered saturated heterocycle which may optionally contain a further heteroatom from the group consisting of S and O or a radical of the formula -NR⁶ and which is optionally substituted by straight-chain or branched alkoxycarbonyl having up to 6 carbon atoms,
in which
R⁶ represents hydrogen or straight-chain or branched alkyl having up to 4 carbon atoms,
and/or the ring systems listed under L are optionally substituted by aryl having 6 to 10 carbon atoms and a 5- to 7-membered aromatic, optionally benzo-fused heterocycle having up to 3 heteroatoms from the group consisting of S, N and O, where these ring systems for their part are optionally substituted up to 2 times by identical or different substituents from the group consisting of halogen, hydroxyl, nitro, carboxyl, trifluoromethyl and straight-chain or branched alkyl, alkoxy, or alkoxycarbonyl having in each case up to 5 carbon atoms, or by a group of the formula -(V')_{b}-NR⁷R⁸,
in which
b has the meaning of a given above and is identical to or different from this meaning,
R⁷ and R⁸ have the meanings of R⁴ and R⁵ given above and are identical to or different from these meanings,
V' has the meaning of V given above and is identical to or different from this meaning,
and their tautomers and salts.

2. Dihydro-[1,2,3]triazolo-[4,5-d]pyrimidin-7-ones according to Claim 1, **characterized in that**
R¹ represents cyclopropyl, cyclobutyl, cyclopentyl, cyclohexyl or cycloheptyl, or
represents a radical of the formula 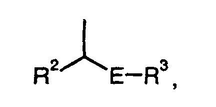 in which
R² represents straight-chain or branched alkyl having up to 8 carbon atoms which is optionally substituted by hydroxyl,
and
E represents a radical of the formula -CH₂-T-,
in which
T represents a straight-chain or branched alkylene chain having up to 8 carbon atoms,
R³ represents hydrogen or phenyl which is optionally substituted up to 2 times by identical or different substituents from the group consisting of fluorine, chlorine, bromine, hydroxyl, nitro, trifluoromethyl and straight-chain or branched alkyl or alkoxy having in each case up to 5 carbon atoms,
A and D represent hydrogen,
or
A represents hydrogen
and
D represents hydroxyl,
or
A and D together represent a radical of the formula =O,
L represents a radical of the formula 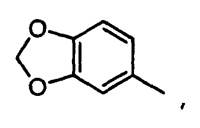 or
represents phenyl, naphthyl, pyridyl, thienyl, indolyl or furyl, which are optionally substituted up to 3 times by identical or different substituents selected from the group consisting of fluorine, chlorine, bromine, trifluoromethyl, hydroxyl, nitro, carboxyl, straight-chain or branched alkyl, alkoxy and alkoxycarbonyl having in each case up to 5 carbon atoms and/or by a radical of the formula -(V)ₐNR⁴R⁵,
in which
a represents a number 0 or 1,
V represents a radical of the formula -CO or -SO₂,
R⁴ and R⁵ are identical or different and represent hydrogen or straight-chain or branched acyl or alkoxycarbonyl having in each case up to 4 carbon atoms, or
represent straight-chain or branched alkyl having in each case up to 5 carbon atoms which is optionally substituted by hydroxyl, amino or by straight-chain or branced alkyl- or dialkylamino having in each case up to 5 carbon atoms,
or
R⁴ and R⁵ together with the nitrogen atom form a morpholinyl, piperidinyl or piperazinyl ring which is optionally substituted via a nitrogen atom by straight-chain or branched alkyl having up to 3 carbon atoms, which rings are optionally substituted by straight-chain or branched alkoxycarbonyl having up to 4 carbon atoms,
and/or the ring systems listed under L are optionally substituted by naphthyl, phenyl, pyridyl, indolyl, thienyl and furyl which are optionally substituted by fluorine, chlorine, bromine, hydroxyl, nitro, carboxyl, trifluoromethyl or by straight-chain or branched alkyl, alkoxy or alkoxycarbonyl having in each case up to 3 carbon atoms or by a group of the formula -(V')_{b}NR⁷R⁸,
in which
b has the meaning of a given above and is identical to or different from this meaning,
V' has the meaning of V given above and is identical to or different from this meaning,
R⁷ and R⁸ have the meanings of R⁴ and R⁵ given above,
and their tautomers and salts.

3. Dihydro-[1,2,3]triazolo-[4,5-d]pyrimidin-7-ones according to Claim 1 or 2, **characterized in that**
R¹ represents cyclopropyl, cyclobutyl, cyclopentyl, cyclohexyl or cycloheptyl, or
represents a radical of the formula 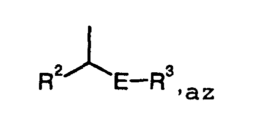 in which
R² represents straight-chain or branched alkyl having up to 7 carbon atoms which is optionally substituted by hydroxyl,
and
E represents a radical of the formula -CH₂-T-,
in which
T represents a straight-chain or branched alkylene chain having up to 7 carbon atoms,
R³ represents hydrogen or phenyl which is optionally substituted up to 2 times by identical or different substituents from the group consisting of fluorine, chlorine, bromine, hydroxyl, nitro, trifluoromethyl and straight-chain or branched alkyl or alkoxy having in each case up to 4 carbon atoms,
A and D represent hydrogen,
or
A represents hydrogen
and
D represents hydroxyl,
or
A and D together represent a radical of the formula =O,
L represents a radical of the formula 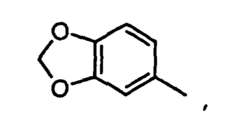 or
represents phenyl or pyridyl which are optionally substituted up to 3 times by identical or different substituents selected from the group consisting of fluorine, chlorine, bromine, trifluoromethyl, hydroxyl, nitro, carboxyl, straight-chain or branched alkyl, alkoxy and alkoxycarbonyl having in each case up to 4 carbon atoms and/or by a radical of the formula -(V)ₐNR⁴R⁵,
in which
a represents a number 0 or 1,
V represents a radical of the formula -CO or -SO₂,
R⁴ and R⁵ are identical or different and represent hydrogen or straight-chain or branched acyl or alkoxycarbonyl having in each case up to 3 carbon atoms, or
represent straight-chain or branched alkyl having in each case up to 4 carbon atoms which is optionally substituted by hydroxyl, amino or by straight-chain or branched alkyl- or dialkylamino having in each case up to 3 carbon atoms,
or
R⁴ and R⁵ together with the nitrogen atom form a morpholinyl, piperidinyl or piperazinyl ring which is optionally substituted via a nitrogen atom by straight-chain or branched alkyl having up to 3 carbon atoms, which rings are optionally substituted by straight-chain or branched alkoxycarbonyl having up to 4 carbon atoms,
and/or the ring systems listed under L are optionally substituted by phenyl or pyridyl,
and their tautomers and salts.

4. Process for preparing the dihydro-[1,2,3]triazolo[4,5-d]pyrimidin-7-ones according to Claims 1 to 3, **characterized in that**, if A and D represent hydrogen, compounds of the general formula (II), 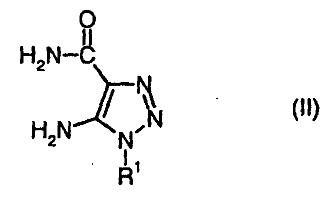 in which
R¹ is as defined above
are reacted with compounds of the general formula (III)
L-CH₂-CO₂R⁹ (III)
in which
L is as defined above
and
R⁹ represents C₁-C₄-alkyl,
in inert solvents, if appropriate in the presence of a base,
and the substituents listed under the substituents R¹, R³ and L are, if appropriate, introduced or derivatized by subsequent reactions such as acylation, oxidation, substitution and/or reductions.

5. Medicaments, comprising one or more dihydro-[1,2,3]triazolo-[4,5-d]pyrimidin-7-ones according to Claims 1 to 3 in combination with customary auxiliaries and excipients.

6. Use of dihydro-[1,2,3]triazolo-[4,5-d]pyrimidin-7-ones according to Claims 1 to 3 in combination with customary auxiliaries and excipients for preparing medicaments.

7. Use of dihydro-[1,2,3]triazolo-[4,5-d]pyrimidin-7-ones according to Claims 1 to 3 in combination with customary auxiliaries and excipients for preparing medicaments for treating cardiovascular disorders, such as, for example, for treating hypertension, neuronal hypertension, stable and unstable angina, peripheral and cardiovascular disorders, arrhythmias, for treating thromboembolic disorders and ischaemias such as mycardial infarction, stroke, transitory and ischaemic attacks, angina pectoris, obstruction of peripheral circulation, prevention of restenoses after thrombolysis therapy, percutaneous transluminal angioplasty (PTA), percutaneous transluminal coronary angioplasties (PTCA) and bypass, and for treating disorders of the urogenital system such as hypertrophy of the prostate, incontinence and, in particular, for treating erectile dysfunction and female sexual dysfunction.

## Revendications

1. Dihydro-[1,2,3]triazolo-[4,5-d]pyrimidine-7-ones de formule générale (I) 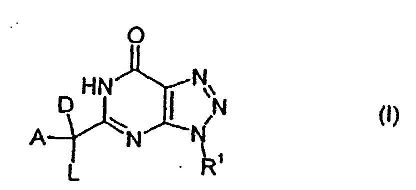 dans laquelle
R¹ est un groupe cycloalkyle ayant 3 à 8 atomes de carbone, ou bien
un reste de formule 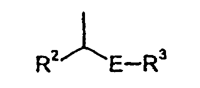 dans laquelle
R² est un groupe alkyle linéaire ou ramifié ayant jusqu'à 10 atomes de carbone, qui est éventuellement substitué par un radical hydroxy,
E représente un groupe de formule -CH₂-T,
dans laquelle
T est une chaîne alkylénique linéaire ou ramifiée ayant jusqu'à 10 atomes de carbone,
R³ représente l'hydrogène ou un groupe aryle ayant 6 à 10 atomes de carbone, qui est éventuellement substitué jusqu'à 3 fois identiques ou différentes par un halogène, un radical hydroxy, nitro, trifluorométhyle ou par un radical alkyle ou alkoxy linéaire ou ramifié ayant chacun jusqu'à 6 atomes de carbone,
A et D représentent l'hydrogène,
ou bien
A est l'hydrogène
et
D est un groupe hydroxy,
ou bien
A et D forment conjointement un reste de formule =O,
L représente un reste de formule 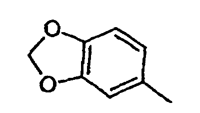
ou bien
un reste aryle ayant 6 à 10 atomes de carbone ou un hétérocycle aromatique pentagonal à heptagonal éventuellement condensé au benzène, ayant jusqu'à 3 hétéroatomes de la série S, N et/ou O, les systèmes cycliques indiqués en L ci-dessus étant substitués 1 à 3 fois identiques ou différentes par un ou plusieurs des substituants suivants : halogène, hydroxy, nitro, trifluorométhyle, carboxy, alkyle, alkoxy et alkoxycarbonyle linéaires ou ramifiés ayant chacun jusqu'à 6 atomes de carbone, un reste de formule -(V)ₐ-NR⁴R⁵,
dans laquelle
a représente le nombre 0 ou 1,
V est un reste de formule -CO ou -SO₂,
R⁴ et R⁵ sont identiques ou différents et représentent l'hydrogène ou un groupe acyle ou alkoxycarbonyle linéaire ou ramifié ayant chacun jusqu'à 6 atomes de carbone, ou bien
un groupe alkyle linéaire ou ramifié ayant jusqu'à 6 atomes de carbone, qui est éventuellement substitué par un radical hydroxy, amino ou par un radical alkyl- ou dialkylamino linéaire ou ramifié ayant chacun jusqu'à 6 atomes de carbone, ou bien
R⁴ et R⁵ forment conjointement avec l'atome d'azote un hétérocycle pentagonal ou hexagonal saturé, qui peut éventuellement contenir un autre hétéroatome de la série S, O ou un reste de formule -NR⁶ et qui est éventuellement substitué par un groupe alkoxycarbonyle linéaire ou ramifié ayant jusqu'à 6 atomes de carbone,
où
R⁶ représente l'hydro ou un groupe alkyle linéaire ou ramifié ayant jusqu'à 4 atomes de carbone,
et/ou les systèmes cycliques énumérés pour L sont éventuellement substitués par un groupe aryle ayant 6 à 10 atomes de carbone et un hétérocycle aromatique pentagonal à heptagonal éventuellement condensé au benzène, ayant jusqu'à 3 hétéroatomes de la série S, N et/ou O, ces systèmes cycliques étant eux-mêmes éventuellement substitués jusqu'à 2 fois identiques ou différentes par des substituants de la série halogène, hydroxy, nitro, carboxyle, trifluorométhyle ou par un groupe alkyle, alkoxy ou alkoxycarbonyle linéaire ou ramifié ayant chacun jusqu'à 5 atomes de carbone ou par un groupe de formule -(V')_{b}-NR⁷R⁸,
dans laquelle
b a la définition indiquée ci-dessus pour a et est égal à a ou en est différent,
R⁷ et R⁸ ont la définition indiquée ci-dessus pour R⁴ et R⁵ et y sont identiques ou en sont différents,
V' a la définition indiquée ci-dessus pour V et y est identique ou en est différent,
et leurs tautomères et les sels.

2. Dihydro-[1,2,3]triazolo-[4,5-d]pyrimidine-7-ones suivant la revendication 1, **caractérisées en ce que**
R¹ est un groupe cyclopropyle, cyclobutyle, cyclopentyle, cyclohexyle ou cycloheptyle, ou bien
un reste de formule 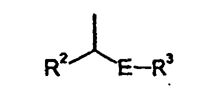 dans laquelle
R² est un groupe alkyle linéaire ou ramifié ayant jusqu'à 8 atomes de carbone, qui est éventuellement substitué par un radical hydroxy,
et
E représente un reste de formule -CH₂-T-,
dans laquelle
T est une chaîne alkylénique linéaire ou ramifiée ayant jusqu'à 8 atomes de carbone,
R³ représente l'hydrogène ou un groupe phényle qui est éventuellement substitué jusqu'à 2 fois identiques ou différentes par du fluor, du chlore, du brome, un radical hydroxy, nitro, trifluorométhyle ou par un radical alkyle ou alkoxy linéaire ou ramifié ayant chacun jusqu'à 5 atomes de carbone,
A et D représentent l'hydrogène,
ou bien
A est l'hydrogène
et
D est un groupe hydroxy,
ou bien
A et D forment ensemble un reste de formule =O,
L est un reste de formule 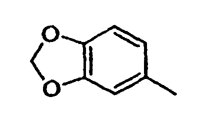
ou bien
des restes phényle, naphtyle, pyridyle, thiényle, indolyle ou furyle qui sont éventuellement substitués jusqu'à 3 fois identiques ou différentes par des substituants choisis dans le groupe fluor, chlore, brome, trifluorométhyle, hydroxy, nitro, carboxy, alkyle, alkoxy et alkoxycarbonyle linéaires ou ramifiés ayant chacun jusqu'à 5 atomes de carbone et/ou par un reste de formule -(V)ₐNR⁴R⁵,
dans laquelle
a représente le nombre 0 ou 1,
V est un reste de formule -CO ou -SO₂,
R⁴ et R⁵ sont identiques ou différents et représentent l'hydrogène ou un groupe acyle ou alkoxycarbonyle linéaire ou ramifié ayant chacun jusqu'à 4 atomes de carbone, ou bien
un groupe alkyle linéaire ou ramifié ayant dans chaque cas jusqu'à 5 atomes de carbone, qui est éventuellement substitué par un radical hydroxy, un radical amino ou par un radical alkyl- ou dialkylamino linéaire ou ramifié ayant dans chaque cas jusqu'à 5 atomes de carbone,
ou bien
R⁴ et R⁵ forment conjointement avec l'atome d'azote un noyau morpholinyle, pipéridinyle ou pipérazinyle qui est éventuellement substitué, par l'intermédiaire d'un atome d'azote, par un radical alkyle linéaire ou ramifié ayant jusqu'à 3 atomes de carbone, et qui sont éventuellement substitués par un radical alkoxycarbonyle linéaire ou ramifié ayant jusqu'à 4 atomes de carbone,
et/ou les systèmes cycliques énumérés pour L sont éventuellement substitués par des radicaux naphtyle, phényle, pyridyle, indolyle, thiényle et furyle qui sont éventuellement substitués par du fluor, du chlore, du brome, un radical hydroxy, nitro, carboxyle, trifluorométhyle ou par un radical alkyle, alkoxy ou alkoxycarbonyle linéaire ou ramifié ayant chacun jusqu'à 3 atomes de carbone ou par un groupe de formule -(V')_{b}NR⁷R⁸,
où
b a la définition indiquée ci-dessus pour a et y est égal ou en est différent,
V' a la définition indiquée pour V et y est identique ou en est différent,
R⁷ et R⁸ ont les définitions indiquées pour R⁴ et R⁵,
et leurs tautomères et les sels.

3. Dihydro-[1,2,3]triazolo-[4,5-d]pyrimidine-7-ones suivant la revendication 1 ou 2, **caractérisées en ce que**
R¹ est un groupe cyclopropyle, cyclobutyle, cyclopentyle, cyclohexyle ou cycloheptyle, ou bien
un reste de formule 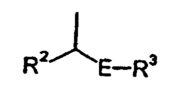 dans laquelle
R² est un groupe alkyle linéaire ou ramifié ayant jusqu'à 7 atomes de carbone, qui est éventuellement substitué par un radical hydroxy,
et
E représente un reste de formule -CH₂-T-,
dans laquelle
T est une chaîne alkylénique linéaire ou ramifiée ayant jusqu'à 7 atomes de carbone,
R³ représente l'hydrogène ou un groupe phényle qui est éventuellement substitué jusqu'à 2 fois identiques ou différentes par du fluor, du chlore, du brome, un radical hydroxy, nitro, trifluorométhyle ou par un radical alkyle ou alkoxy linéaire ou ramifié ayant dans chaque cas jusqu'à 4 atomes de carbone,
A et D représentent l'hydrogène,
ou bien
A est l'hydrogène
et
D est un groupe hydroxy,
ou bien
A et D forment ensemble un reste de formule =O,
L est un reste de formule 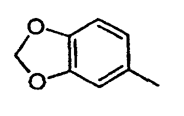
ou bien
un reste phényle ou un reste pyridyle qui est substitué jusqu'à 3 fois identiques ou différentes par des substituants choisis dans le groupe fluor, chlore, brome, trifluorométhyle, hydroxy, nitro, carboxy, alkyle, alkoxy et alkoxycarbonyle linéaires ou ramifiés ayant chacun jusqu'à 4 atomes de carbone et/ou par un reste de formule -(V)ₐNR⁴R⁵,
dans laquelle
a représente le nombre 0 ou 1,
V est un reste de formule -CO ou -SO₂,
R⁴ et R⁵ sont identiques ou différents et représentent l'hydrogène ou un groupe acyle ou alkoxycarbonyle linéaire ou ramifié ayant dans chaque cas jusqu'à 3 atomes de carbone, ou bien
un groupe alkyle linéaire ou ramifié ayant dans chaque cas jusqu'à 4 atomes de carbone, qui est éventuellement substitué par un radical hydroxy, amino ou par un radical alkyl- ou dialkylamino linéaire ou ramifié ayant dans chaque cas jusqu'à 3 atomes de carbone,
ou bien
R⁴ et R⁵ forment conjointement avec l'atome d'azote un noyau morpholinyle, pipéridinyle ou pipérazinyle qui est éventuellement substitué par l'intermédiaire d'un atome d'azote, par un radical alkyle linéaire ou ramifié ayant jusqu'à 3 atomes de carbone, et qui sont éventuellement substitués par un radical alkoxycarbonyle linéaire ou ramifié ayant jusqu'à 4 atomes de carbone,
et/ou les systèmes cycliques indiqués pour L sont éventuellement substitués par un reste phényle ou pyridyle,
et leurs tautomères et les sels.

4. Procédé de production des dihydro-[1,2,3]triazolo[4,5-d]pyrimidine-7-ones suivant les revendications 1 à 3,
**caractérisé en ce que** lorsque A et D représentent l'hydrogène, on fait réagir des composés de formule générale (II) 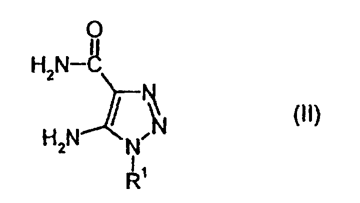 dans laquelle
R¹ a la définition indiquée ci-dessus,
avec des composés de formule générale (III)
L-CH₂-CO₂R⁹ (III)
dans laquelle
L a la définition indiquée ci-dessus
et
R⁹ est un groupe alkyle en C₁ à C₄,
dans des solvants inertes, éventuellement en présence d'une base
et on introduit, le cas échéant, les substituants indiqués comme substituants R¹, R³ et L par des réactions subséquentes telles qu'acylation, oxydation, substitution et/ou réductions, c'est-à-dire qu'on effectue une dérivatisation.

5. Médicaments contenant une ou plusieurs dihydro-[1,2,3]triazolo-[4,5-d]pyrimidine-7-ones suivant les revendications 1 à 3 en association avec les substances auxiliaires et les supports classiques.

6. Utilisation de dihydro-[1,2,3]triazolo-[4,5-d]-pyrimidine-7-ones suivant les revendications 1 à 3 en association avec les substances auxiliaires et les supports classiques pour la préparation de médicaments.

7. Utilisation de dihydro-[1,2,3]triazolo-[4,5-d]-pyrimidine-7-ones suivant les revendications 1 à 3 en association avec les substances auxiliaires et les supports classiques pour la préparation de médicaments destinés au traitement de maladies cardiovasculaires, par exemple pour le traitement de l'hypertension artérielle, de l'hypertonie neuronale, de l'angor stable et instable, de troubles vasculaires périphériques et cardiaques, d'arythmies, pour le traitement de maladies thrombo-emboliques et d'ischémies telles que l'infarctus du myocarde, l'apoplexie cérébrale, des attaques transitoires et ischémiques, l'angine de poitrine, des troubles de la circulation périphérique, pour empêcher des resténoses après une thrombolyse, une angioplastie transluminale percutanée (PTA), une angioplastie coronaire transluminale percutanée (PTCA) et un pontage ainsi que pour le traitement de maladies du système urogénital telles qu'une hypertrophie de la prostate, l'incontinence et en particulier pour le traitement de troubles de l'érection, et d'un dysfonctionnement sexuel chez la femme.
